# EUROPEAN PATENT APPLICATION

(11) **EP 4 230 713 A1**
(43) Date of publication of application: **23.08.2023**
(21) Application number: 23164459.2
(22) Date of filing: 18.04.2017
(51) Int. Cl.: C11B 9/02, A23L 33/105, A61K 36/185, A23L 27/10, C11B 9/00, B01D 11/02

(54) **ISOLATION OF PLANT EXTRACTS**

(30) Priority: 18.04.2016 US 201662324154 P
(62) Divisional of application: 17786496.4
(71) Applicant: Gene Pool Technologies, Inc., Cherry Hills Village, CO 80111 (US)
(72) Inventor: MORROW, Kenneth, Oakland, 94612 (US)
(74) Representative: FRKelly

(57) **Abstract**

A plant extract can be produced from a plant material, such as a cannabis plant material, via an ambient temperature distillation followed by a cold trap condensation. The cold trap apparatus has at least one sealable chamber under vacuum connected to a condenser for extracting chemical compositions at an un-elevated temperature from a plant material. Desirable chemicals can be volatilized under negative pressure at low temperatures utilizing the cold trap system described herein.

## Description

### RELATED APPLICATIONS

This application claims priority under 35 U.S.C. §119(e) to U.S. Provisional Patent Application No. 62/324,154, filed April 18, 2016, which is incorporated herein by reference.

### BACKGROUND

Plants contain substantial amounts of different classes of chemicals. Many of these chemicals have been extracted, isolated and characterized. One common plant extract is called an essence oil. Essence oils (sometimes referred to as "essential oils", "volatile oils", "ethereal oils", "aetherolea" or the "oil of the plant" from which they are extracted) are some of the oldest plant derivatives known to man. An essence oil is a concentrated hydrophobic liquid or solid containing volatile aroma compounds extracted from a plant, which captures a characteristic fragrance of the plant from which it is derived. Each species of plant can have a unique essence oil profile. Essence oils have been used for millennia in medicine (*e.g*., sandalwood oil), food and drink (*e.g.,* Balsam of Peru oil), cleaning/disinfecting agents (*e.g.,* eucalyptus oil), perfumes (*e.g.,* lavender oil), beauty treatment (*e.g.,* cedarwood oil), and religious ceremony (*e.g*., Myrrh oil), to name a few areas. One common use is in alternative medicine. For example, essence oils are frequently used in aromatherapy (*e.g*., chamomile oil), where they can be volatilized or diluted in a carrier oil and used in massage, diffused in the air by a nebulizer, heated over a candle flame (vaporized), or burned as incense.

Essence oils are complex mixtures of naturally occurring organic chemicals extracted from a plant material. Common classes of chemicals found in essence oils and other plant extracts are terpenes and terpene derivatives (*e.g*., terpenoids), many of which are aromatic. Terpenes and terpene derivatives can be found in many plants, such as in the cannabis plant. Terpenes are part of a large group of volatile unsaturated hydrocarbons found in essence oils of plants, including cannabis (with over 120 terpenes), which play a large part in the aromas and flavors of plants. Examples include hemiterpenes, monoterpenes, sesquiterpenes, diterpenes, sesterterpenes, triterpenes, sesquarterpenes, tetraterpenes, polyterpenes and norisoprenoids. Cannabinoids are a class of terpenophenolic (part terpenoid, part phenol) compounds. While terpenes are hydrocarbon groups created by various combinations of the isoprene units that make them up, and may be aromatic, all phenols are aromatic hydrocarbons, which means they have a very pronounced aroma. Terpenoids are compounds related to terpenes, but may also include other functional groups (*e.g*., hydroxyls and/or carbonyls) or have their molecular framework rearranged; the terms are often used interchangeably. Cannabinoids, being part-terpene and part-phenol, have pronounced aromas and flavors.

Isolation of essence oils and other plant extracts has been achieved through a number of different methods, such as, distillation *(e.g.,* steam), solvent *(e.g.,* alcohol) extraction, absolute oil extraction, expression, resin tapping, and cold pressing. In addition to essence oils, other chemical constituents can be extracted from a plant. For example, in steam distillation, a plant is soaked with water in a vessel, which is then heated to generate steam. As the temperature rises in the vessel, the glands of the plant burst and release their oils and other chemical constituents of the plant into the water used in the process. When the steam is generated, it can be passed through a condenser to collect the essence oils, while the remaining water can contain plant hydrosols.

There are numerous limitations to conventional processes for extracting and isolating essence oils and other chemical constituents from a plant. For example, in solvent extraction, many undesirable chemicals can be extracted with the desired chemicals. It is challenging to find a solvent or series of solvents that can efficiently and safely extract desired chemicals contained in the plant, while minimizing or preventing the extraction of undesirable chemicals contained in the plant. Another limitation with conventional distillation processes is that the application of heat to effect distillation and separation of the chemicals can destroy or degrade many desirable chemicals in the plant.

Accordingly, there is a need for an improved process to extract chemical compositions from a plant material, such as a cannabis plant, without destroying or degrading desired chemicals contained in the plant material, while minimizing or preventing the extraction of undesirable chemicals contained in the plant material. The present invention is useful because it allows one to extract and isolate terpenes, terpene derivatives, and other chemicals found in plant materials that previously have been challenging, if not impossible, to isolate. This provides a beneficial use, particularly, with regard to the isolation of extracts from the cannabis plant.

### SUMMARY

One aspect of the disclosure provides a method of producing an extract from a plant material, the method comprising:
(a) placing the plant material in a compartment at a temperature of less than about 35°C;
(b) applying positive or negative pressure to the compartment at a temperature of less than about 35°C to volatilize chemical compounds contained in the plant material;
(c) transporting the volatilized chemical compounds via a conduit to a condenser comprising at least one sealable chamber;
(d) condensing the volatilized chemical compounds in the condenser to form the extract; and
(e) collecting the extract from the condenser.

Another embodiment of the disclosure provides a novel cold trap system (*e.g*., method and apparatus) for extracting and isolating terpenes, terpene derivatives, and other chemicals from a plant material, such as a cannabis plant. In embodiments, the cold trap system can comprise at least one (*e.g*., cryogenic) condenser comprising a sealable chamber having a bottom, an air or liquid intake and an air or liquid outtake. A plant material can be placed in a compartment, to which positive or negative pressure (*e.g.*, a vacuum) can be applied at an unelevated temperature (*e.g*., at ambient or cooler temperatures) to volatilize desired chemicals of the plant material, which are then condensed in the sealable container of the condenser to form a plant extract. The resulting extract can then be collected. In embodiments where the plant is cannabis, a liquid or solid condensed extract of cannabis can be produced. Volatilizing and condensing a cannabis plant in the cold trap system at unelevated (*e.g*., low) temperatures can avoid the degradation and destruction of many chemicals. The resulting extract can be enriched with desirable terpene and terpene derivatives.

Another aspect of the disclosure provides a novel method for producing, isolating and collecting one or more novel plant extracts from a plant material in the cold trap system described herein. The plant material can be distilled and volatilized at low temperatures (*e.g.*, less than about 35 °C) under positive or negative pressure and the resulting volatilized compounds can be condensed to form the novel plant extract(s). Since the distillation and condensation are accomplished at low temperatures, many heat sensitive chemicals will not be degraded or destroyed in the resulting extract. In some embodiments, a series of volatilization steps may be performed with incremental increasing or decreasing temperatures to form new extracts with desirable chemicals. In certain embodiments, a series of condensation steps can be performed at progressively lower or higher temperatures. Accordingly, the cold trap system may comprise one or more of a series of sealable chambers of differing temperatures to capture one or more plant extracts containing different chemical compositions. For example, the first chamber can be run at a low temperature, and subsequent chambers can be run at decreased or increased temperature ranges to produce a series of extracts. The plant extracts may be collected, further purified and/or formulated through conventional methods to make them suitable for their end uses.

### BRIEF DESCRIPTION OF THE DRAWINGS

The following drawings form part of the specification and are included to further demonstrate certain embodiments or various aspects of the invention. In some instances, embodiments of the invention can be best understood by referring to the accompanying drawings in combination with the detailed description presented herein. The description and accompanying drawings may highlight a certain specific example, or a certain aspect of the invention. However, one skilled in the art will understand that portions of the example or aspect may be used in combination with other examples or aspects of the invention. The following drawings illustrate certain embodiments and aspects according to some embodiments of the disclosure.
**FIG. 1** shows a schematic representation illustrating a plant extraction method utilizing a cold trap system.
**FIG. 2** shows a front view of a cold trap cooling chamber.
**FIG. 3** shows a front view of a series of cold trap cooling chambers.
**FIG. 4** shows a front view of another type of cold trap cooling chamber.
**FIG. 5** shows a top view of a single chamber cold trap system.
**FIG. 6** shows a front view of an unassembled single chamber cold trap system.
**FIG. 7** shows a front view of a refrigeration system for use in a cold trap system.
**FIG. 8** shows a schematic representation illustrating two different cold trap systems (**A** and **B**).
**FIG. 9** shows a top view of a cold trap system.
**FIG. 10** shows a front view of a cold trap system.

### DETAILED DESCRIPTION

Terpenes and terpene derivatives are a large class of organic compounds produced from a variety of plant materials (*e.g*., shrubs, herbs, flowers, etc.), particularly conifers. Terpenes and terpenoids are the primary constituents of the essential oils of many types of plants and flowers. Essential oils are used widely as fragrances in perfumery, and in medicine and alternative medicines such as aromatherapy. Synthetic variations and derivatives of natural terpenes and terpenoids also greatly expand the variety of aromas used in perfumery and flavors used in food additives.

As used herein, the term "terpene" means an organic compound built on an isoprenoid structural scaffold or produced by combining isoprene units. Often, terpene molecules found in plants may produce aromas.

The structures of terpenes are built with isoprenes, which are 5 carbon structures. Flavonoids are generally considered to be about 15 carbon structures with two phenyl rings and a heterocyclic ring. There can be an overlap in which a flavonoid could be considered a terpene. However, not all terpenes could be considered flavonoids.

Within the context of this disclosure, the term terpene includes hemiterpenes, monoterpenols, terpene esters, diterpenes, monoterpenes, polyterpenes, tetraterpenes, terpenoid oxides, sesterterpenes, sesquiterpenes, norisoprenoids, or their derivatives.

Derivatives of terpenes include terpenoids in their forms of hemiterpenoids, monoterpenoids, sesquiterpenoids, sesterterpenoid, sesquarterpenoids, tetraterpenoids, triterpenoids, tetraterpenoids, polyterpenoids, isoprenoids, and steroids. They may have various different forms: alpha, beta, gamma, oxo-isomers, or combinations thereof. Examples of terpenes within the context of this disclosure include: 7,8-dihydroionone, acetanisole, acetic acid, acetyl cedrene, anethole, anisole, benzaldehyde, bergamotene (alphacis-bergamotene) (alpha-trans-bergamotene), bisabolol (beta-bisabolol), borneol, bornyl acetate, butanoic/butyric acid, cadinene (alpha-Cadinene) (gamma-cadinene), cafestol, caffeic acid, camphene, camphor, capsaicin, carene (delta-3-Carene), carotene, carvacrol, carvone, dextro-carvone, laevo-carvone, caryophyllene (beta-Caryophyllene), caryophyllene oxide, castoreum absolute, cedrene (alpha-Cedrene) (beta-Cedrene), cedrene epoxide (alpha-Cedrene Epoxide), cedrol, cembrene, chlorogenic acid, cinnamaldehyde (alpha-amyl-Cinnamaldehyde), (alpha-hexyl-cinnamaldehyde), cinnamic acid, cinnamyl alcohol, citronellal, citronellol, cryptone, curcumene (alpha-curcumene) (gamma-curcumene), decanal, dehydrovomifoliol, diallyl disulfide, dihydroactinidiolide, dimethyl disulfide, eicosane/icosane, elemene (beta-elemene), estragole, ethyl acetate, ethyl cinnamate, ethyl maltol, eucalyptol/1,8-cineole, eudesmol (alpha-eudesmol) (beta-eudesmol) (gammaeudesmol), eugenol, euphol, farnesene, farnesol, fenchol (beta-fenchol), fenchone, geraniol, geranyl acetate, germacrenes, germacrene B, guaia-1(10), 11-diene, guaiacol, guaiene (alphaguaiene), gurjunene (alpha-gurjunene), herniarin, hexanaldehyde, hexanoic acid, humulene (alpha-humulene) (beta-humulene), ionol (3-oxo-alpha-ionol) (beta-ionol), ionone (alphaionone) (beta-ionone), ipsdienol, isoamyl acetate, isoamyl alcohol, isoamyl formate, isoborneol, isomyrcenol, isopulegol, isovaleric acid, isoprene, kahweol, lavandulol, limonene, gamma-linolenic acid, linalool, longifolene, alpha-longipinene, lycopene, menthol, methyl butyrate, 3-mercapto-2-methylpentanal, mercaptan/thiols, beta-mercaptoethanol, mercaptoacetic acid, allyl mercaptan, benzyl mercaptan, butyl mercaptan, ethyl mercaptan, methyl mercaptan, furfuryl mercaptan, ethylene mercaptan, propyl mercaptan, thenyl mercaptan, methyl salicylate, methylbutenol, methyl-2-methylvalerate, methyl thiobutyrate, myrcene (beta-myrcene), gamma-muurolene, nepetalactone, nerol, nerolidol, neryl acetate, nonanaldehyde, nonanoic acid, ocimene, octanal, octanoic acid, p-cymene, pentyl butyrate, phellandrene, phenylacetaldehyde, phenylethanethiol, phenylacetic acid, phytol, pinene, beta-pinene, propanethiol, pristimerin, pulegone, quercetin, retinal, rutin, sabinene, sabinene hydrate, cis-sabinene hydrate, trans-sabinene hydrate, safranal, alpha-selinene, alphasinensal, beta-sinensal, beta-sitosterol, squalene, taxadiene, terpin hydrate, terpineol, terpine-4-ol, alpha-terpinene, gamma-terpinene, terpinolene, thiophenol, thujone, thymol, alphatocopherol, tonka undecanone, undecanal, valeraldehyde/pentanal, verdoxan, alpha-ylangene, umbelliferone, and/or vanillin.

In an embodiment, the cold trap system comprises at least one sealable chamber attached to a vacuum apparatus. The chamber comprises a bottom, an air (or liquid) intake and an air (or liquid) outtake. The chamber may have surfaces or baffles and cooling means capable of sufficiently regulating low temperatures inside the chamber. Any cryogenic means may be used to maintain a low temperature in the sealed chamber. For example, coolants, such as liquid nitrogen, dry ice in acetone, or other similar solvents can be used to cool surfaces or baffles of the chamber. Any method of refrigeration that maintains low temperature control can be used in the disclosure. In certain embodiments, the sealed cooling chamber is kept at a freezing or near-freezing temperature to produce desirable enriched extracts. Optionally, the chamber contains a valve at the bottom. The chamber also optionally contains a plurality of refrigerated condensing plates, coils or baffles. The chamber may contain or be connected to a collector having non-absorbent, absorbent, non-adsorbent, or adsorbent properties.

Wet or dry plant material can be used. The plant material may be raw, fresh, preserved, dried, cut, trimmed, macerated, milled, screened, or any combination thereof. Optionally, the plant material may first be processed into a wet or dry concentrate. The method disclosed herein may be used to prepare an extract from a plant material. The extract may be called a botanical drug substance (BDS), which is defined in the Guidance for Industry Botanical Drug Products Draft Guidance by the US Department of Health and Human Services, Food and Drug Administration Center for Drug Evaluation and Research (August 2000) as a drug substance derived from one or more plants, algae, or macroscopic fungi. The botanical raw materials can be subjected to one or more of the following processes: pulverization, decoction, expression, aqueous extraction, ethanolic extraction, or other similar processes. "Plant material" is defined as a plant or plant part (*e.g*., bark, wood, leaves, stems, roots, flowers, fruits, seeds, berries or parts thereof) as well as exudates thereof. In some embodiments, the plant material is derived from one or more cannabis plants. The term "cannabis" encompasses all types of cannabis, including wild type Cannabis sativa, Cannabis chemovars, Cannabis indica, Cannabis ruderalis and variants thereof.

In embodiments, wet or dry plant material may be placed directly in a compartment (*e.g*., chamber) connected to a vacuum source, where chemical constituents of the plant material are volatized at low temperatures (*e.g*., less than about 35 °C) and transferred to a condenser. A solvent extraction need not be performed. Alternatively, in some embodiments, one or more solvent extractions may first be performed to form a concentrate. For example, wet or dry plant material may be mixed into one or more solvents, and the resulting solution/suspension is then filtered and/or evaporated off, and the resulting concentrate is placed in the compartment, which is then subjected to negative or positive pressure at an unelevated temperature to volatize the chemicals contained in the concentrate. The volatilized chemicals are then condensed in one or more sealable chambers to form one or more plant extracts and the resulting plant extracts are then collected. One or more solvents, together or sequentially, may be used to prepare the concentrate. Optionally, the concentrate is winterized prior to being placed in the compartment.

In a particular embodiment, wet or dry plant material is dissolved in a first solvent for a period of time to produce a first solution, optionally, in a dark environment. Any solid matter present in the solvate is filtered off to produce a first filtrate. A second solvent is added to the first filtrate to produce a second solution. The first solvent is evaporated off and the second solution is chilled for a period of time. Any solid matter present in the chilled second solution is filtered out to produce a second filtrate. The second solvent is then evaporated off. The process can be repeated as many times as desired, for example, third, fourth and fifth filtrates can be prepared. The final filtrate is then placed in a vacuum drying apparatus at ambient temperature, for example, at a temperature of less than or equal to 35 °C. The vacuum drying apparatus is a device capable of creating a vacuum (*e.g*., to create positive or negative pressure) and comprises a compartment that may be, optionally, heated via microwaves, ultrasonic waves, conduction heat, convection heat, or some combination thereof. In certain embodiments, the vacuum applies negative pressure to the compartment. The vacuum drying apparatus can be connected to a condenser comprising at least one sealable cooling chamber, having a bottom, and means (*e.g*., port or valve) for the ingress and egress of air (or liquid), optionally, containing a valve at the bottom, and optionally, containing a plurality of refrigerated condensing plates, coils or baffles. The cooling chamber(s) may be connected to at least one collector having non-absorbent, absorbent, non-adsorbent, or adsorbent properties. The cooling chamber(s) (*e.g*., cold trap) is capable of regulating the temperature inside the chamber. The filtrate may be volatilized in the compartment after being subjected to vacuum drying at ambient temperature, and the cooling chamber(s) condenses the volatilized filtrate to form an extract of the plant, which may then be deposited into the collector.

Optionally, any one or more of the filtrates can be *winterized* before being placed in the vacuum drying apparatus. Winterization of cannabis is an effective refinement procedure to remove undesirable lipids (fats, waxes, etc.) and solvents from an extract. In general, winterization consists of soaking a cannabis concentrate (extract, oil or tincture) in alcohol and freezing it in order to precipitate out and remove undesirable chemicals. As disclosed below, cannabis concentrates can be made by solvent extraction with CO₂, nitrogen, butane or other solvents. This process leads to separation of cannabinoids and terpenes from the cannabis plant material, and sometimes produces unwanted chemical substances, such as, plant waxes, fats and chlorophyll. Choosing the right solvents and process parameters can minimize or avoid the extraction of undesirable lipids. Sometimes, simply re-dissolving the concentrate in a suitable solvent(s) and filtering out *(e.g.,* using a coffee or laboratory filter, cheesecloth or strainer) the insoluble chemicals can remove the undesirable chemicals. However, sometimes it is also desirable to perform winterization, especially when the initial solvent extraction process utilized non-polar solvents, such as butane and hexane.

Winterization involves taking the cannabis concentrate produced by solvent extraction and re-dissolving it in another solvent, such as an alcohol or alkane (e.g., 95% ethanol or hexane). Usually, an alcohol is heated *(e.g.,* to about 50 °C) to ensure all the chemicals are in solution. The alcohol may be partially or substantially evaporated off and the resulting solution can be frozen for at least 24 hours at a minimum temperature of about 0 °C *(e.g.,* 48 hours at -18 °C). The lipids contained therein will coagulate and precipitate out of the frozen solution. After this step, the alcohol solution can be filtered to remove the undesirable lipids and the solvent can be substantially evaporated or vacuumed off, leaving a purer product. The extraction and refinement steps can be repeated several times until the desired purity of the product is obtained. Winterization, however, does not generally remove the chlorophyll present in the plant material, which can impart a green hue to the final product. Chlorophyll can be removed via other chemical separation means, such as, washing the concentrate with ethanol tincture or exposing it to sunlight or UV radiation. A disadvantage of winterization is that some of the terpenes and terpene derivatives are also filtered out with the lipids. Another disadvantage is that some desirable terpenes or terpene derivatives may be degraded or destroyed during the heating process. Thus, winterization may not be beneficial if it desired to obtain a more flavorful and aromatic oil product or an oil product containing desirable terpenes that would be lost in the process. Winterization is more generally utilized when high potency cannabinoid extracts are desired.

If winterization is performed, the resulting winterized composition (*e.g*., botanical drug substance) can be allowed to warm to the temperature of the vacuum drying apparatus. In some embodiments, the temperature of the vacuum drying apparatus may be greater than about 35 °C. However, in other embodiments, where desired chemicals are sensitive to high temperatures, the temperature is no warmer than about 35 °C, 30 °C, 25 °C or 20 °C. The resulting extract can be collected as a solid or a liquid. The collected extract can be further separated through conventional means. Fractions can be collected together or individually. The method can be repeated as many times as desired. In some embodiments, the vacuum drying apparatus can be heated via microwaves, ultrasonic waves, convection, conduction, or a combination of ultrasonic waves, microwaves, convection, and/or conduction. In certain embodiments, low or no heat is provided to avoid degradation of heat sensitive chemicals.

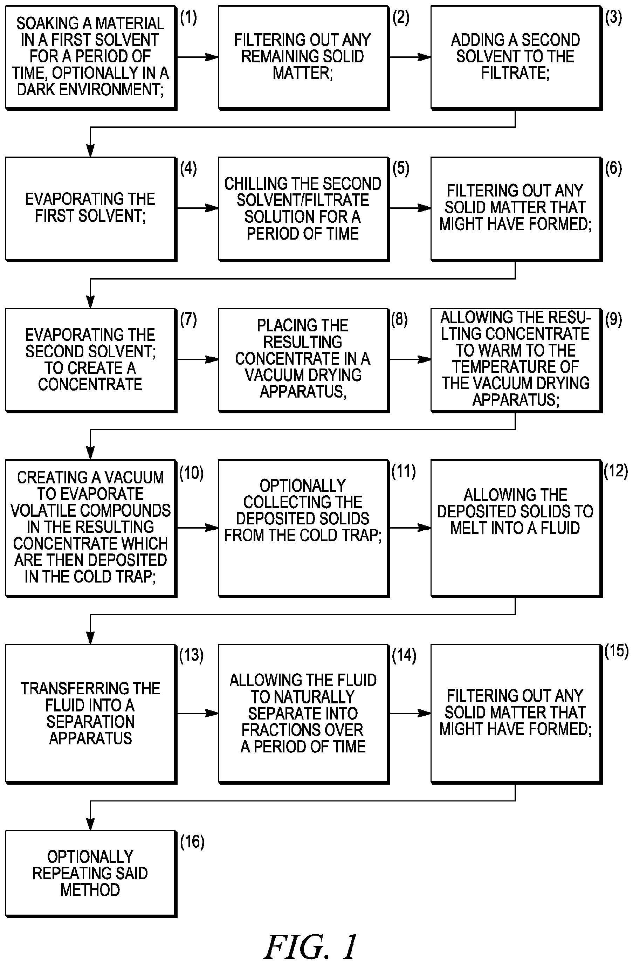

A graphical representation of an embodiment of the disclosure is shown in **FIG. 1****.** Wet or dry plant material can be mixed in at least one solvent (*e.g*., butane or ethanol) and extraction can be performed in a closed loop extractor or by supercritical (*e.g*., CO₂) extraction. For example, the wet or dry material can be macerated and soaked in a first solvent to form a first solvate and, optionally, allowed to sit in a dark environment for a period of time (*e.g*., up to about 2 hours) **1.** The first solvate can be poured through a filter to separate out undesired compounds from the solvent **2.** A second solvent can be added to the first solvate **3.** In certain embodiments, the second solvent generally amounts to about 10% to 200% of the first solvent on a volume-to-volume basis. In particular embodiments, the second solvent amounts to about 25% to 175%, 50% to 150%, or 75% to 125%, of the first solvent. The first solvent can be evaporated off and the essential oils and other chemical constituents contained therein migrate to the second solvent to form a second solvate **4.** In some embodiments, the second solvate can then be placed in a sealable container and put into a freezer *(e.g.,* at about -35°C) and chilled for a period of time *(e.g.,* about 24 hrs.) **5.** The chilled second solvate can then be poured through a filter *(e.g.,* a coffee filter) to recover a concentrate **6.** This process is called winterization and eliminates undesired chemical compounds, such as fats and waxes. The second solvent may then be separated out from the winterized concentrate via conventional means. For example, the concentrate can be poured into a dish *(e.g.,* glass bowl) and placed in front of a fan to evaporate off the second solvent and any residual water **7.** A rotary evaporator can also be utilized to accomplish evaporation.

Once the solvent/water has been evaporated, the concentrate is usually viscous. It can then be placed into a vacuum drying oven at ambient (*e.g*., room) temperature **8.** The oven has a vacuum pump containing a port connected to at least one cooling chamber as described herein. In some embodiments, liquid nitrogen can be utilized to maintain low temperatures in the cooling chamber (*e.g*., cold trap). In one aspect, the oven can be pre-warmed to a temperature of about 20 °C to about 25 °C. The concentrate may also be pre-warmed to about the same temperature of the oven **9.** When the desired temperatures have been achieved, the vacuum pump can be turned on to apply negative pressure **10.** The essence oil and other chemical constituents of the plant material evaporate under the applied vacuum and condense in the cooling chamber (*e.g*., cold trap) **10.** Because the process is run under controlled low temperatures in a vacuum, delicate aromatic chemical compounds will not be destroyed or degraded by heat. Extraction, evaporation, distillation and condensation can be run at controlled low temperatures to preserve desirable chemicals. Alternatively, controlled higher temperatures may be utilized to provide different chemical compositions. Once condensed, the deposited solids may be collected from the cold trap **11** and allowed to melt into a fluid form **12.** The melted fluid may be transferred into a separation apparatus (*e.g*., separatory funnel) **13,** in which the fluid will naturally separate into fractions over a period of time (*e.g.* a few minutes or a few hours) **14.** It may be advantageous to keep the separation apparatus at a low temperature (*e.g*., at or below about 0 °C). Any solid matter that may form can be filtered out **15.** In embodiments, the process may be repeated one or more times **16.**

The cold trap (cooling chamber or condenser) of the invention can take different forms. Several forms are shown in the figures.

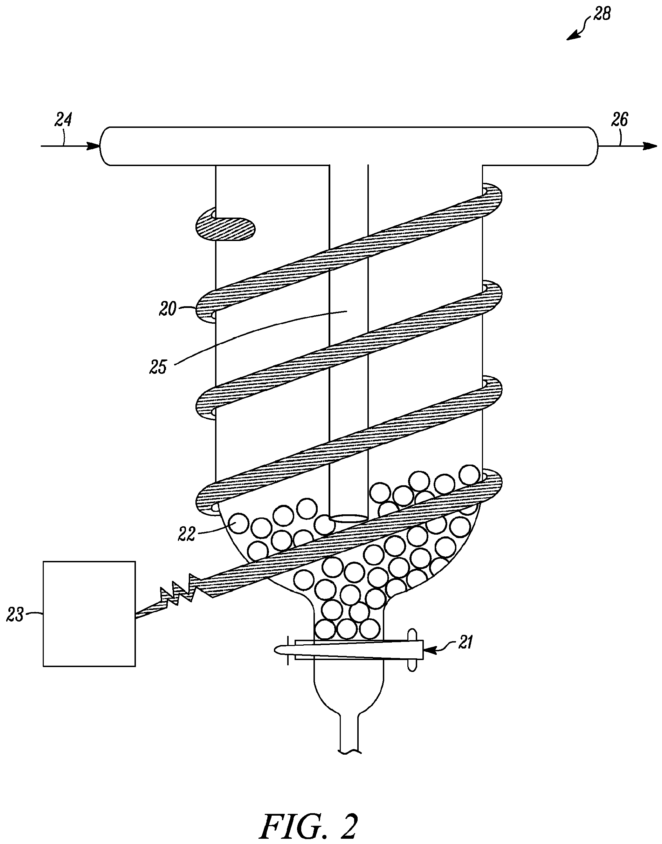

**FIG. 2** shows a front view of a single cooling chamber cold trap system **28.** It comprises an air inlet **24,** an air outlet **26,** cooling/refrigerant coils **20** surrounding a conduit **25,** and a valve **21.** Balls or spheres **22** (*e.g.,* steel balls) having absorbent, non-absorbent, adsorbent or non-adsorbent properties are placed in the bottom of the chamber to increase surface area. The cooling/refrigerant coils **20** are connected to a temperature controller **23.** After a plant material is volatilized by subjecting it to negative pressure in a vacuum oven, the resulting vapor enters the cold trap system **28** through the air inlet **24** and travels down the conduit **25** surrounded by the cooling/refrigerant coils **20,** where it condenses. Any non-condensed vapor can leave the chamber through the air outlet **26.**

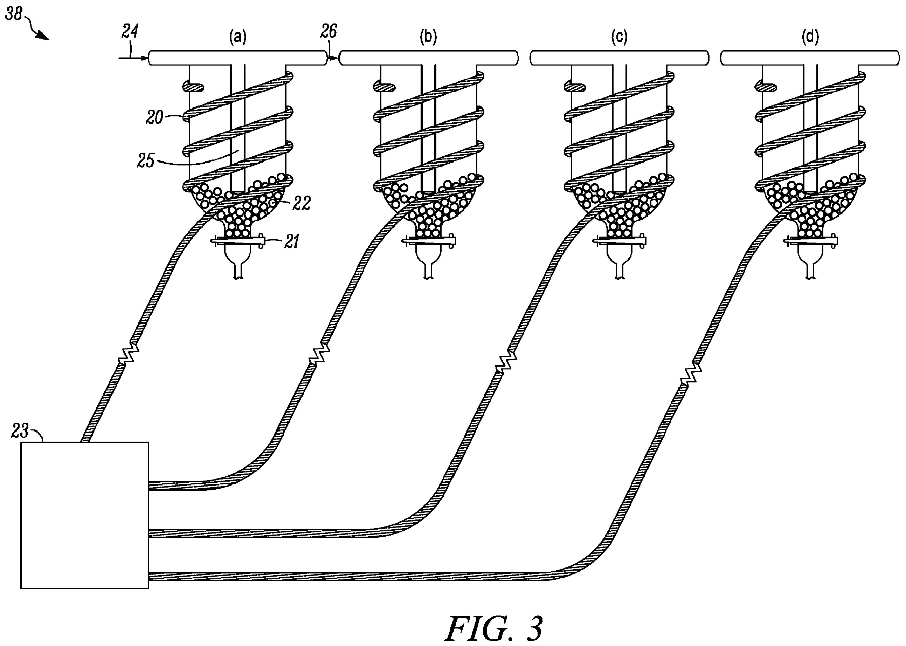

**FIG. 3** shows a front view of a cold trap system **38** in which a series of four single cooling chambers **a, b, c** and **d** are linked together. Each chamber comprises an air inlet **24,** an air outlet **26,** cooling/refrigerant coils **20** surrounding a conduit **25,** a valve **21** and balls or spheres **22** (*e.g.*, steel balls) having absorbent, non-absorbent, adsorbent or non-adsorbent properties in the bottom of the chamber to increase surface area. Each set of cooling/refrigerant coils **20** are connected to the same or different temperature controllers **23.** After a plant material is volatilized by subjecting it to negative pressure in a vacuum oven at a temperature of less than about 35 °C, the resulting vapor enters the cold trap system **29** through the air inlet **24** of the first cooling chamber **a** and travels down the conduit **25** surrounded by the cooling/refrigerant coils **20,** where it condenses. Any non-condensed vapor can leave the first cooling chamber **a** through the air outlet **26,** which is connected to the second cooling chamber **b.** The non-condensed vapor transfers to the second cooling chamber **b.** The same process run in the first cooling chamber **a** is repeated in the second **b,** third **c** and fourth **d** chambers. The four cooling chambers **a, b, c** and **d** can be maintained at different temperatures (*e.g*., at progressively colder temperatures) so that different chemical compositions can be formed and collected.

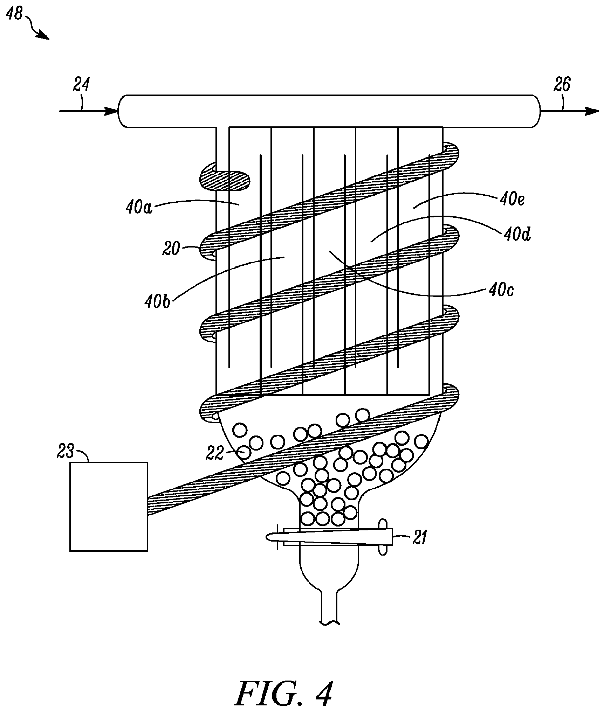

**FIG. 4** shows a front view of a single cooling chamber cold trap system **48** having multiple conduits **40a, 40b, 40c, 40d** and **40e** surrounded by cooling/refrigerant coils **20** connected to a temperature controller **23.** It further comprises an air inlet **24,** an air outlet **26,** a valve **21** and balls or spheres **22** (*e.g*., steel balls) having absorbent, non-absorbent, adsorbent or non-adsorbent properties in the bottom of the chamber to increase surface area. Having multiple conduits **40a, 40b, 40c, 40d** and **40e** can provide for more efficient condensation. The condensed solids may be collected in the cold trap systems **28, 38** and **48,** allowed to melt into a fluid form, and transferred via the valve **21** into a separation apparatus (*e.g*., separatory funnel), in which the fluid will naturally separate into fractions over a period of time (*e.g.* a few minutes or a few hours).

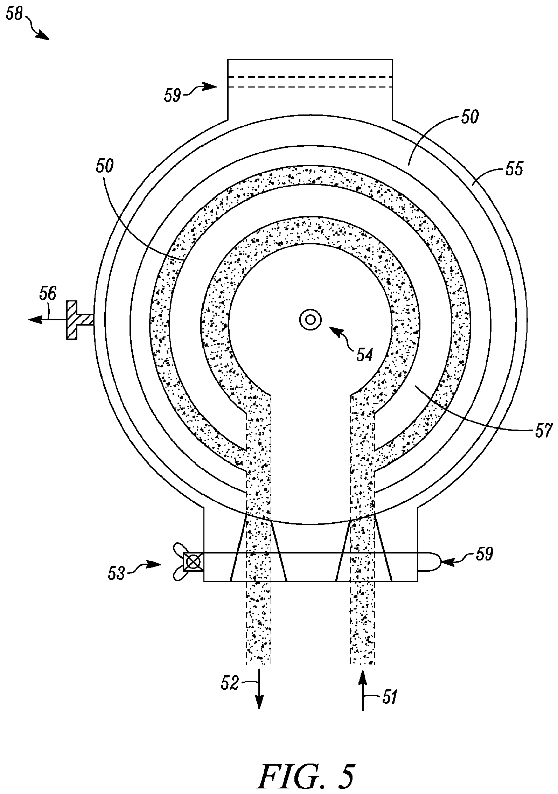

**FIG. 5** shows a top view of a single cold trap system **58.** The volatilized vapor enters though an air inlet **54** and the non-condensed vapor exits through the air outlet **56.** The coolant/refrigerant coils **50** maintain the low temperatures required to effect condensation. Conduits **51** and **52,** respectively, are shown for passing the cooling/refrigerant in and out of the system. A collector bowl or plate **57** is located inside the system to collect the condensed product. A seal or gasket **55** is provided to maintain pressure and prevent leaking in the system. Optionally, the system can be hinged **59** and/or clamped **53** on one or both ends of the apparatus. The chamber can be set at different temperatures to capture and separate different extracts.

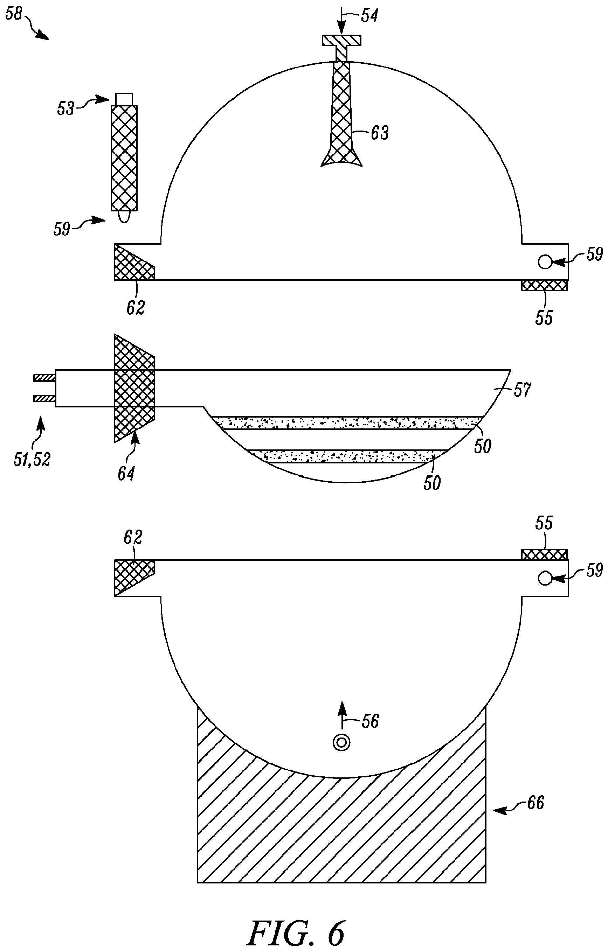

**FIG. 6** shows a front view of a single cold trap system **58.** The volatilized vapor enters though an air inlet **54** and the non-condensed vapor exits through the air outlet **56.** The air inlet may be adjustable **63.** The coolant/refrigerant coils **50** maintain the low temperatures required to effect condensation. Conduits **51** and **52,** respectively, are shown for passing the cooling/refrigerant in and out of the system. A collector bowl or plate **57** is located inside the system to collect the condensed product. A seal or gasket **55** is provided to maintain pressure and prevent leaking in the system. Optionally, the system can be hinged **59** and/or clamped **53** on one or both ends of the apparatus. Pass through cut outs **62** can be located on the top and bottom half of the system. A pass-through plug **64** can be placed in the middle of the apparatus. Optionally, the apparatus can be situated on a stand **66.** The chamber can be set at different temperatures to capture and separate different extracts.

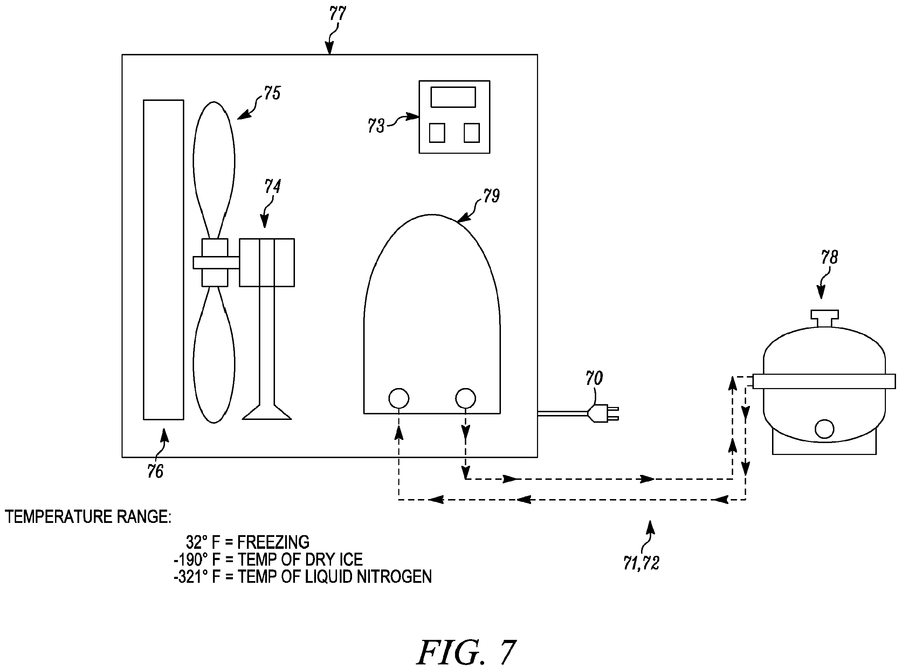

**FIG. 7** shows a side view of one type of refrigeration system **77** for use in a cold trap system **78** (*e.g*., terpene isolator). Lines with arrows are drawn to show the path of refrigerant/coolant **71** and **72** in and out or to and from a controller and pump. The refrigeration system **77** comprises electronic switches **73** to control the temperature and power, a motor **74,** a fan **75** and an evaporator **76.** A pump **79** can be used to push refrigerant/coolant throughout the cold trap system. The refrigeration system can be plugged **70** into an electrical power source. Three cold temperature ranges are shown: 32°F (freezing), -190°F (dry ice) and -321°F (liquid nitrogen). However, various different cold temperatures can also be achieved via use of refrigerants, coolants and/or electronic means, such as, 0 °F, -25 °F, -50 °F, -75 °F, -100 °F, -125 °F and -150 °F.

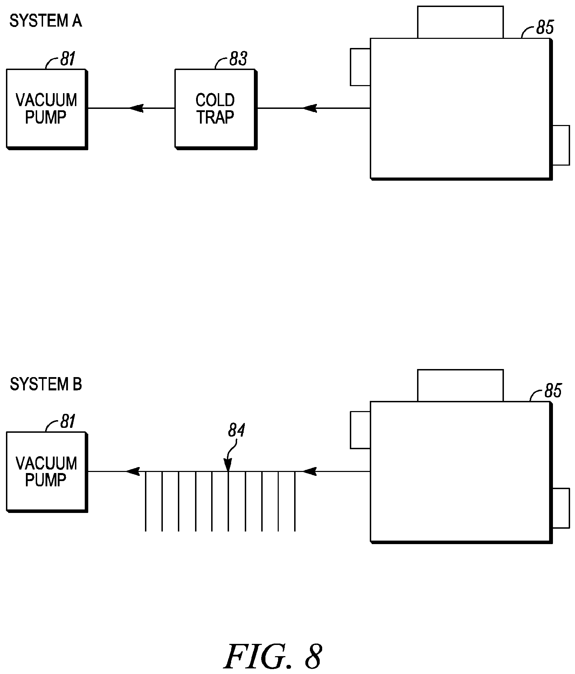

**FIG. 8** shows a schematic representation of two different embodiments (systems **A** and **B**) of a cold trap system. The first embodiment (system **A**) is a single-chamber cold trap system **83** connected to a vacuum pump **81** and a compartment **85.** The second embodiment (system **B**) shows a multi-chamber cold trap system **84** connected to a vacuum pump **81** and a compartment **85.** The compartment can be a drying oven, freeze dryer, rotary evaporator, sealed chamber or the like. In embodiments, the temperature of compartment is less than about 35 °C. The drying ovens can be fitted with the capability to apply microwaves, ultrasonic waves, conduction heat, convection heat, or a combination thereof. Wet or dry cannabis plant material, or a concentrate thereof, can be placed in the compartment. An applied vacuum can create negative pressure to effect volatilization of vapor compositions comprising terpenes and terpene derivatives, which are then condensed and collected in the chamber of the cold trap systems.

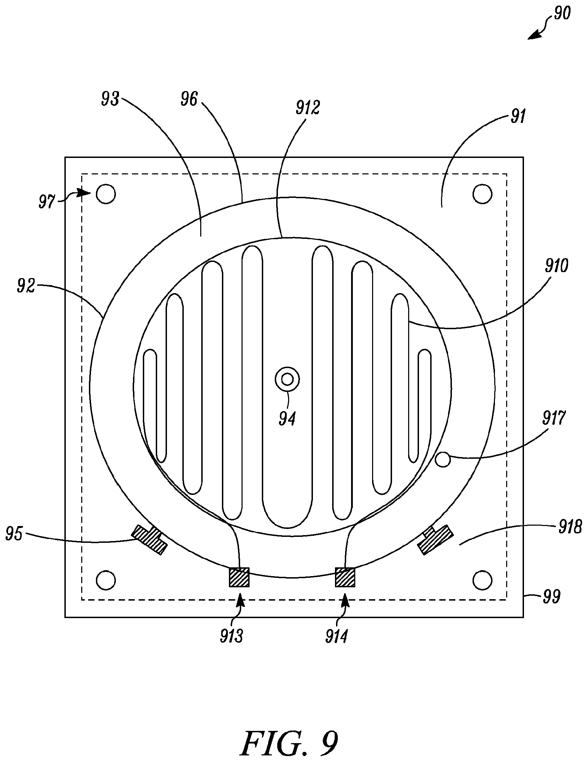

**FIG. 9** shows a top view of a cold trap system **90.**

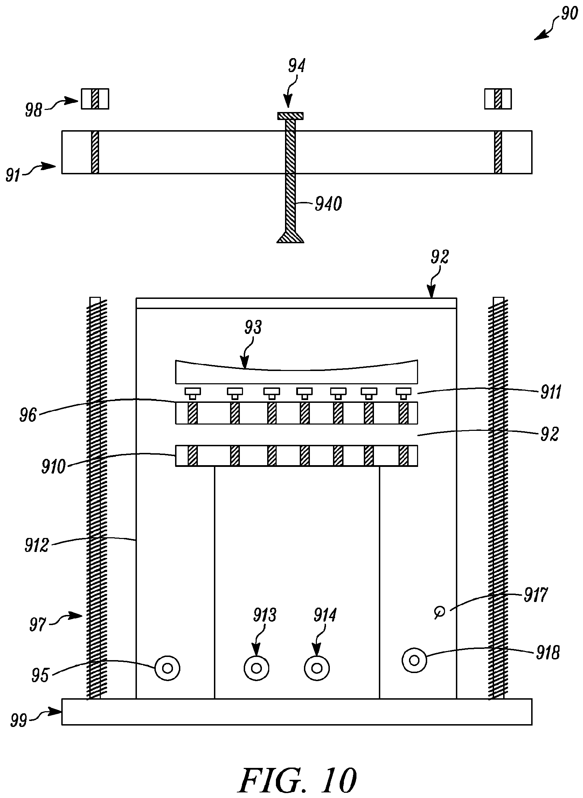

**FIG. 10** shows a front view of a cold trap system **90.**

The following elements are shown in the cold trap systems of **FIGS. 9** and/or **10:**
- **91**: Lexan^{™} plexiglass top;
- **92**: Rubber gasket (or weld to seal);
- **93**: Collector dish (removable);
- **94**: Air inlet;
- **95**: Air outlet;
- **96**: Cooling plate;
- **97**: Threaded rod;
- **98**: Threaded nut;
- **99**: Base;
- **910**: Cooling/refrigerant coils;
- **911**: Bolts/fasteners (or weld to seal);
- **912**: Vacuum chamber housing;
- **913**: Coolant/refrigerant inlet to cooling coils;
- **914**: Coolant/refrigerant outlet to cooling coils;

- **917**: Valve for purging oxygen;
- **918**: Nitrogen gas inlet; and
- **940**: Adjustable distance air inlet.

In an embodiment, low temperature distillation of a plant material volatilizes into a vapor comprising air, water, terpenes and terpene derivatives (*e.g*., extracted from a cannabis plant). The vapor enters the air inlet **94.** Any vapor that enter the vacuum chamber housing **912** is cooled and condensed/frozen to a removable collector dish **93** in the form of ice and/or liquid. Non-condensed vapor leaves through an air outlet **95.** Optionally, the air inlet is adjustable **940.** The collector dish **93** is cooled by coolant/refrigerant that circulates in/out **(913/914)** or cools from within the cooling plate **96** by coolant/refrigerant contained in the cooling/refrigerant coils **910.** The vapor source can be generated from the vacuum chamber housing **912** (any vacuum chamber, rotary evaporator, freeze dryer or sealed vessel with, optionally, microwave or ultrasonic assistance). Nitrogen gas can be utilized through the inlet **918** via a valve **917** to purge any oxygen in the system and minimalize the possibility of undesired terpene oxidation. The apparatus can include a Lexan^{™} plexiglass top **91,** rubber gasket(s) (or weld to seal) **92,** threaded rod(s) **97,** and threaded nut(s) **98.** The bottom of the apparatus is the base **99.**

In some embodiments, when volatilized chemicals exit the exhaust port of the vacuum oven, they can be captured in the cooling chamber in the form of ice containing the condensed chemicals. The ice can be periodically scraped/collected from the cooling chamber and placed in a sealed container. Once accumulated, the mixture of chemicals, solvent and water that form the ice can be left at room temperature to melt the ice. The resulting fluid can then be poured into a separatory funnel or graduated cylinder. Some of the chemicals will rise to the top of the funnel or cylinder and form an amber (yellow or golden) colored layer. The amber layer contains desirable terpenes and terpene derivatives. Other chemicals separate out below this layer and form a clear layer. The clear layer may comprise a hydrosol. The mixture can be allowed to sit in a sealed separatory funnel or graduated cylinder for an appropriate amount of time to effect separation (*e.g*., about 24 hrs.) at room temperature or in a reduced temperature environment (*e.g*., a refrigerator or freezer) to accelerate separation. The separated layers can be drawn out from the graduated cylinder by using a pipette or by turning a drain valve at the bottom of the separatory funnel. The separated layers can be moved into separate vessels. Note that the color of the chemical compositions can change depending on the variations in the process conditions and chemicals recovered. Differing temperature and process variations can affect the color of the final extract. For example, the amber color can vary from darker to lighter shades, and even approach a clear liquid. Similarly, the clear hydrosols can also take on color depending on the temperature and other parameters of the process.

One particular embodiment provides a method for isolating plant extracts (*e.g*., oils and/or hydrosols) comprising terpenes, terpene derivatives and/or other chemical compounds from a cannabis plant material via vacuum or short-path steam distillation, specifically, capturing the chemical compounds in the cold trap system described herein after they pass through and are volatilized in a vacuum drying oven or any other vacuum apparatus under negative or positive pressure at a temperature less than about 35 °C. Optionally, the cannabis plant material can be winterized before it is vacuum or short-path steam distilled taking care when delicate, heat-sensitive chemical compositions are desired. In embodiments, the vacuum drying oven can be run under low pressure to create negative pressure. The extraction process utilizes the selectivity of the solvent and can produce clear and colored (*e.g*., amber or golden) extracts that are substantially free of waxes and fats. Because the cold trap extraction process is conducted under controlled low temperatures, it will produce little to no thermal degradation of desirable products. The products may be useful as a fragrance, flavoring, pesticide, fungicide, or pharmaceutical composition. The products may be used alone or in vaporizers, edibles, drinks, and the like. They may be used as a flavor booster during the growing or drying of plant material. Medical uses include treatments of cancer, psychological disorders, eating disorders, and the like.

A series of chambers or cold trap systems of differing temperatures can be used. The chemical compounds can condense at varying different low temperatures in a cold trap system described herein, thus allowing for fractionation/separation without utilizing excessive heat. In contrast, traditional methods, such as rotary vacuum distillation, utilize higher temperatures that can destroy lighter and/or more delicate volatile compounds. In one embodiment, the fractionation/separation technique described herein can utilize some minimum heat combined with varying low (*e.g*., sub-zero) temperatures by having a plurality of separate cooling chambers of differing temperatures, beginning with the least cooled chamber and progressing to the coldest chamber or vice-a-versa. There can be two, three, four or more different chambers. The chambers can be cooled electronically, by utilization of liquid nitrogen or by other known means.

In certain embodiments, the vacuum apparatus applies negative pressure to effect volatilization of the chemicals. In certain embodiments, the multi-chamber cold trap system progresses from the least cooled chamber to the coldest chamber.

A concentrate (or extract) of a plant material refers to a substance obtained by extracting a chemical composition from a plant material with a solvent and then substantially removing the solvent from the chemical composition. In some embodiments, the process of extracting a chemical composition using a solvent includes a hot or cold solvent extraction. A variety of solvents may be utilized to obtain a plant extract. The solvents may be polar, nonpolar, protic, aprotic, or a combination thereof. Generally, organic solvents (*e.g*., hydrocarbons and alcohols) work well. Suitable hydrocarbons include small chain alkanes (*e.g*., propane, butane, pentane, and hexane) and small chain alcohols (*e.g*., methanol, ethanol, n-propanol, and iso-propanol). Other suitable solvents include chloroform, ether, limonene, water, acetonitrile, ethyl acetate, and toluene. In general, a broad range of solvents may be utilized, such as: carbon dioxide, hydrogen, neon, nitrogen, argon, methane, ethane, propane, ammonia, water, xenon, methanol, ethanol, 1-propanol, 2-propanol, 1-hexanol, 2-methoxy ethanol, tetrahydrofuran, 1,4-dioxane, acetonitrile, methylene chloride, dichloroethane, chloroform, ethyl acetate, propylene carbonate, N,N-dimethylacetamide, dimethyl sulfoxide, formic acid, carbon disulfide, acetone, toluene, hexanes, pentanes, trifluoromethane, nitrous oxide, sulfur hexafluoroide, butane, isobutane, ethyl ether, benzotrifluoride, (p-chlorophenyl) trifluoromethane, chlorofluorocarbon (CFC), hydrofluorocarbon, HFA-134a, terpenes, terpenoids, or a combination thereof. Exemplary terpenes and terpenoids include limonene, myrcene, linalool, alpha bisabolol, delta-3 carene, borneol, alpha-pinene, beta-pinene, beta-caryophyllene, alpha-humulene, eucalyptol, terpineol, caryophyllene, cineole, isoprene, prenol, isovaleric acid, geraniol, terpineol, humulene, farnesenes, farnesol, cafestol, kahweol, cembrene, taxadiene, geranylfarnesol, squalene, lycopene, gamma-carotene, alpha-carotene, beta-carotene, polyisoprene, gutta-percha, megastigmane-3,9-diol, 3-oxo-7,8-dihydro-alpha-ionol, and the like.

In embodiments, the plant material can be placed in a container (or vessel) and subjected to a solvent extraction to form a concentrate comprising a crude plant extract. This is often called a maceration step. During bulk maceration, the plant material can be suspended in the solvent and left to sit for an appropriate period of time (i.e., "the sitting phase"). The ratio of plant material to solvent may vary considerably. For example, the weight ratio of plant material: solvent may be about 1:10, 1:7.5, 1:5, 1:2.5 or 1:1. The sitting time may be several hours, several days, several weeks, or several months, depending on the desired potency or purpose of the maceration. Generally, macerating several minutes, several hours or several days will suit most purposes. While soaking in the solvent, components of the plant material (e.g., glandular trichomes) can begin to soften as the solvent wets, infuses and steeps the plant material. During maceration, components of the plant material can begin to separate out. The maceration step can be generally run at room temperature; however, it may also be conducted at a reduced or increased temperature.

A number of steps can be taken to hasten a maceration step and increase its efficiency. For example, prior to the maceration step, the plant material may be pulverized to a fine powder or otherwise grinded to allow for faster infusion of the solvent. Furthermore, prior to a maceration step, the plant material may also be dried to avoid any undesirable solvent or environmental reactions. Moreover, one may actively (intermittently or continuously) shake, stir (*e.g*., kinetic maceration) or mix a suspension of plant material and solvent. In another step, one may gently heat the plant material, solvent or suspension of plant material and solvent or place it in a warm sunny place. However, when macerating a cannabis plant, heating or exposing the suspension to sunlight may not be recommended when it contains volatile and heat-sensitive desirable compounds that can become degraded when subjected to the heat or sunlight. Accordingly, in some embodiments, the maceration step can be conducted in a cool, dark and closed environment. A skilled operator can perform one or more of the above steps to quicken the maceration process and increase the quality of the crude plant extract produced by the process.

Once the plant material has been macerated, one may desire to filter off plant particulates in the suspension (*e.g*., decoction) to further refine the crude plant extract. Depending on the quantity and quality of the plant material and solvent used in the process, a small to a large amount of undesirable plant particles may be present in the suspension. While not necessary, filtering off residual plant particulates can enhance the quality of the product. Numerous filtration methods may be applied, such as a strainer, fine mesh sieve, muslin cloth or cheesecloth. The maceration process may be repeated once, twice or several times with fresh solvent. Optionally, the last residue of extract may be pressed out of the plant particles using a mechanical press or a centrifuge.

In certain embodiments, the macerating step of extracting a crude plant extract in a solvent as described herein is conducted in conjunction with a conventional extraction process, such as an ultrasound-assisted solvent extraction, hot solvent extraction, cold solvent extraction, percolation extraction, Soxhlet extraction, pressurized solvent extraction, reflux and steam distillation extraction, and supercritical fluid extraction (SFE) or fractional supercritical fluid extraction (FSFE). Supercritical fluid extraction is an extraction wherein a fluid at a temperature and pressure above its critical point is employed, or a fluid above its critical temperature, regardless of pressure, is employed. Below the critical point, the fluid can coexist in both gas and liquid phases, but above the critical point there is only one phase.

Equipment and techniques for carrying out supercritical fluid extraction are known to those skilled in the art. See, McHugh, M. And Krukonis, V., Supercritical Fluid Extraction, 2nd ed, Butterworth-Heinemann, Boston, 1994; Johnston, K. P., Penninger, J. M. L., Supercritical Fluid Science and Technology, ACS Symposium Series 406, American Chemical Society, Washington, D.C.; and Taylor, L. T., Supercritical Fluid Extraction, John Wiley & Sons, New York, 1996. As used herein, "supercritical fluid extraction" or "SFE" refers to the process of separating one or more components (extractant) from another (matrix) using supercritical fluids as the extracting solvent. Extraction is usually from a solid matrix (*e.g*., cannabis plant material), but can also be from liquids or resinous material (*e.g*., hash oil). Although numerous supercritical fluids can be used, carbon dioxide (CO₂) is the most commonly used supercritical fluid, sometimes modified by co-solvents, such as ethanol or methanol. Extraction conditions for supercritical fluids are generally above the critical temperature and critical pressure of the desired fluid. Addition of modifiers may slightly alter this.

In certain embodiments, the supercritical fluid extraction can be carried out multiple times. In such embodiments, the supercritical fluid extraction is a fractional supercritical fluid extraction. "Fractional supercritical fluid extraction" (or "FSFE") refers to a supercritical fluid extraction that is carried out multiple times. Each time the supercritical fluid extraction is carried out, at least one of the (i) solvent system (with respect to polarity and proticity), (ii) temperature and (iii) pressure will vary. For example, in reference to the cannabis plant, the FSFE can include a supercritical fluid extraction that is carried out two times, three times, four times, etc. For each fraction, the temperature, pressure, period of time, and supercritical fluid solvent system are each independently selected *(e.g.,* can independently be the same or different).

Prior to an initial supercritical fluid extraction of a cannabis plant material, the plant material may be dried of any water present. Such drying may increase the efficiency of the supercritical fluid extraction, or may lower the cost of shipping and storage of cannabis plant material. Drying may also decarboxylate the cannabis plant material. In embodiments, there is no need to decarboxylate the plant material. The cannabis plant material may be air-dried or dried at an elevated temperature with or without reduced pressure (e.g., in vacuo). However, similar to a hot solvent extraction process, the application of elevated temperatures to a cannabis plant material must be carefully applied to avoid the degradation or destruction of desirable chemicals. Specifically, the cannabis may be dried in a vacuum at an elevated temperature. Machines capable of drying cannabis are known in the art and include an oven, or similar device, such as a rotating air drum drier. For use in the processes of this disclosure, the temperature at which the cannabis may be dried can be greater than about 25 °C. Specifically, the temperature can be greater than about 30 °C, greater than about 40 °C, or greater than about 50 °C. In some embodiments, the temperature can be greater than about 60 °C, greater than about 70 °C, or greater than about 80 °C.

Supercritical fluid extraction employs a solvent (and optionally, a co-solvent), which possesses physical properties suitable as a supercritical fluid. Suitable solvents (and/or co-solvents) useful in supercritical fluid extraction are disclosed, for example, in McHugh, M. and Krukonis, V., Supercritical Fluid Extraction, 2nd ed, Butterworth-Heinemann, Boston, 1994; Johnston, K. P., Penninger, J. M. L., Supercritical Fluid Science and Technology, ACS Symposium Series 406, American Chemical Society, Washington, D.C.; and Taylor, L. T., Supercritical Fluid Extraction, John Wiley & Sons, New York, 1996. Suitable exemplary solvents (and co-solvents) useful in supercritical fluid extraction include: carbon dioxide, hydrogen, neon, nitrogen, argon, methane, ethane, propane, ammonia, water, xenon, methanol, ethanol, 1-propanol, 2-propanol, 1-hexanol, 2-methoxy ethanol, tetrahydrofuran, 1,4-dioxane, acetonitrile, methylene chloride, dichloroethane, chloroform, ethyl acetate, propylene carbonate, N,N-dimethylaceamide, dimethyl sulfoxide, formic acid, carbon disulfide, acetone, toluene, hexanes, pentanes, trifluoromethane, nitrous oxide, sulfur hexafluoroide, butane, isobutane, ethyl ether, benzotrifluoride, (p-chlorophenyl) trifluoromethane, chlorofluorocarbon (CFC), hydrofluorocarbon, HFA-134a, terpenes, terpenoids, or a combination thereof. Exemplary terpenes and terpenoids include limonene, myrcene, linalool, alpha bisabolol, delta-3 carene, borneol, alpha-pinene, beta-pinene, beta-caryophyllene, alpha-humulene, eucalyptol, terpineol, caryophyllene, cineole, isoprene, prenol, isovaleric acid, geraniol, terpineol, humulene, farnesenes, farnesol, cafestol, kahweol, cembrene, taxadiene, geranylfarnesol, squalene, lycopene, gamma-carotene, alpha-carotene, beta-carotene, polyisoprene, gutta-percha, megastigmane-3,9-diol, 3-oxo-7,8-dihydro-alpha-ionol, and the like.

Typically, when present, the solvent and/or co-solvent will be present in about 1 wt. % to about 50 wt. %, in about 1 wt. % to about 30 wt. %, or in about 1 wt. % to about 10 wt. % of the supercritical fluid solvent system. Many different solvent systems may be used to carry out SPE. Classes of solvents include: (i) a polar protic solvent system, (ii) a polar aprotic solvent system, (iii) a nonpolar protic solvent system, (iv) a nonpolar aprotic solvent system, or (v) a combination thereof.

Examples of SFE and FSFE can be found in US Patent Number 9,186,386, which is incorporated herein in its entirety. Furthermore, sub-critical fluid extraction and fractional sub-critical fluid extraction can also be utilized. Examples of sub-critical fluid extraction can be found in US Patent Number 7,344,736, which is incorporated herein in its entirety.

Novel extracts may be obtained by the methods described herein. While the extract can be obtained directly upon removal of the solvent, it can also subsequently be further purified by standard purification techniques. Alternatively, the extract or a further purified fraction thereof, can be used directly in a pharmaceutical dosage form (*e.g*., transdermal patch or oral thin film). The processes described herein can be run multiple times. Multiple extracts can be recovered, discarded or mixed together depending on the end use. One or more extracts can be combined or further purified by conventional purification methods, such as, chromatography, adsorption, crystallization, distillation, liquid-liquid extraction, filtration, fractional distillation, precipitation, recrystallization, sublimation, or a combination thereof.

By utilizing unelevated low temperatures (in at least one extraction process), the cold trap system and methods described herein can produce novel extracts enriched with desirable terpenes and terpene derivatives that heretofore were difficult or impossible to obtain. In some embodiments, an extraction process can be run at an unelevated low temperature to produce a unique extract product enriched with terpene and/or terpene derivatives from the starting material. Performing the process under vacuum also helps to avoid oxidation and degradation of desirable terpenes and/or terpene derivatives. Subsequent extraction processes may be run on the unique extract product, with or without the application of heat, to capture multiple unique terpene (and/or terpene derivative) enriched extracts.

In some embodiments, subsequent extraction processes can be run contemporaneously, in parallel, or in a series, at differing low temperatures to capture desirable terpenes and/or terpene derivatives. One embodiment can run an extraction process at an unelevated low temperature, followed by a series of subsequent extractions at slowly ascending (or descending) temperatures. Using freezing or near freezing condenser apparatuses can also help provide the capture of unique chemicals. In summary, the conventional way to perform extraction by applying heat can degrade and/or destroy desirable terpenes and terpene derivatives, especially the lighter chemicals. In contrast, the cold trap system described herein avoids chemical degradation or destruction of desirable chemicals and can produce extracts containing unique enrichments of desirable terpenes and/or terpene derivatives.

The level of enrichment of desirable chemicals in the final extract may be small, moderate or large, up to and including substantially pure desirable terpenes and/or terpene derivatives. Depending on the requirements of the final product, one can design the cold trap system to produce a final product having desirable properties.

The extract obtained by the processes described herein for cannabis plant material can include at least one of the following compounds: cannabinol (CBN), cannabinolic acid (CBNA), DELTA (9)-tetrahydrocannabinol (DELTA.(9)-THC), DELTA (9)-tetrahydrocannabinolic acid (DELTA (9)-THCA), DELTA (9)-cannabidiol (DELTA (9)-CBD), DELTA (9)-tetrahydrocannabidiolic acid (DELTA (9)-CBDA), DELTA (8)-tetrahydrocannabinol (DELTA (8)-THC), DELTA (8)-tetrahydrocannabinolic acid (DELTA (8)-THCA), DELTA (8)-tetrahydrocannabidiol (DELTA (8)-CBD), DELTA (8)-tetrahydrocannabidiolic acid (DELTA (8)-CBDA), DELTA (9)-tetrahydrocannabivarin (DELTA (9)-THV), cannabigerol (CBG), cannabigerolic acid (CBGA), cannabichromene (CBC), cannabichromenic acid (CBCA), cannabicyclol (CBL), cannabicyclolic acid (CBLA), cannabivarin (CBV), tetrahydrocannabivarin (THCV), cannabidivarin (CBDV), cannabichromevarin (CBCV), cannabigerovarin (CBGV), cannabigerol monomethyl ether (CBGM), beta.-caryophyllene epoxide, mentha-1,8(9)-dien-5-ol, pulegone, limonene, limonene oxide, alpha-terpinene, terpinen-4-ol, carvacrol, carvone, 1,8-cineole, p-cymene, fenchone, pulegone-1,2epoxide, beta-myrcene, cannaflavin A, and cannaflavin B.

The extracts obtained by the processes described herein for the cannabis plant can be formulated into a pharmaceutical dosage form containing any one or more of the above compounds. The extracts can be formulated for the use of effective amounts of the compositions described herein for use in medical therapy. The extracts can also be formulated for the use of effective amounts of the composition as described herein for the manufacture of a medicament to treat a disease in a mammal, for example, cancer in a human. The medicament can include a pharmaceutically acceptable diluent, excipient, or carrier. The extracts can also be formulated for other uses, including homeopathic remedies, flavorants, aroma therapy, and the like.

### Definitions

The following definitions are included to provide a clear and consistent understanding of the specification and claims. As used herein, the recited terms have the following meanings. All other terms and phrases used in this specification have their ordinary meanings as one of skill in the art would understand.

References in the specification to "one embodiment", "an embodiment", etc., indicate that the embodiment described may include a particular aspect, feature, structure, moiety, or characteristic, but not every embodiment necessarily includes that aspect, feature, structure, moiety, or characteristic. Moreover, such phrases may, but do not necessarily, refer to the same embodiment referred to in other portions of the specification. Further, when a particular aspect, feature, structure, moiety, or characteristic is described in connection with an embodiment, it is within the knowledge of one skilled in the art to affect or connect such aspect, feature, structure, moiety, or characteristic with other embodiments, whether or not explicitly described.

The singular forms "a," "an," and "the" include plural reference unless the context clearly dictates otherwise. Thus, for example, a reference to an extract or a chemical composition includes a plurality of such extracts or chemical compositions, so that an extract X includes a plurality of extracts X. It is further noted that the claims may be drafted to exclude any optional element. As such, this statement is intended to serve as antecedent basis for the use of exclusive terminology, such as "solely," "only," and the like, in connection with any element described herein, and/or the recitation of claim elements or use of "negative" limitations.

The term "and/or" means any one of the items, any combination of the items, or all of the items with which this term is associated. The phrase "one or more" is readily understood by one of skill in the art, particularly when read in context of its usage.

The term "about" can refer to a variation of ± 5%, ± 10%, ± 20%, or ± 25% of the value specified. For example, "about 50" percent can in some embodiments carry a variation from 45 to 55 percent. For integer ranges, the term "about" can include one or two integers greater than and/or less than a recited integer at each end of the range. Unless indicated otherwise herein, the term "about" is intended to include values, e.g., weight percentages, proximate to the recited range that are equivalent in terms of the functionality of the individual ingredient, the composition, or the embodiment. The term about can also modify the end-points of a recited range as discuss above in this paragraph.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible sub-ranges and combinations of sub-ranges thereof, as well as the individual values making up the range, particularly integer values. A recited range (e.g., weight percentages or carbon groups) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc. As will also be understood by one skilled in the art, all language such as "up to", "at least", "greater than", "less than", "more than", "or more", and the like, include the number recited and such terms refer to ranges that can be subsequently broken down into sub-ranges as discussed above. In the same manner, all ratios recited herein also include all sub-ratios falling within the broader ratio. Accordingly, specific values recited for radicals, substituents, and ranges, are for illustration only; they do not exclude other defined values or other values within defined ranges for radicals and substituents.

One skilled in the art will also readily recognize that where members are grouped together in a common manner, such as in a Markush group, the invention encompasses not only the entire group listed as a whole, but each member of the group individually and all possible subgroups of the main group. Additionally, for all purposes, the invention encompasses not only the main group, but also the main group absent one or more of the group members. The invention therefore envisages the explicit exclusion of any one or more of members of a recited group. Accordingly, provisos may apply to any of the disclosed categories or embodiments whereby any one or more of the recited elements, species, or embodiments, may be excluded from such categories or embodiments, for example, for use in an explicit negative limitation.

The term "contacting" refers to the act of touching, making contact, or of bringing to immediate or close proximity, including at the cellular or molecular level, for example, to bring about a physiological reaction, a chemical reaction, or a physical change, e.g., in a solution, in a reaction mixture, *in vitro,* or *in vivo.*

### Medical Treatment

An "effective amount" refers to an amount effective to treat a disease, disorder, and/or condition, or to bring about a recited effect. For example, an effective amount can be an amount effective to reduce the progression or severity of the condition or symptoms being treated. Determination of a therapeutically effective amount is well within the capacity of persons skilled in the art, especially in light of the detailed disclosure provided herein. The term "effective amount" is intended to include an amount of an extract or chemical composition described herein, or an amount of a combination of extracts or chemical compositions described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" can extend to prophylaxis and can include prevent, prevention, preventing, lowering, stopping or reversing the progression or severity of the condition or symptoms being treated. As such, the term "treatment" can include medical, therapeutic, and/or prophylactic administration, as appropriate.

### Pharmaceutical Formulations

The extracts or chemical compositions produced by the cold trap system described herein can be used to prepare therapeutic pharmaceutical compositions, for example, by combining the extracts or chemical compositions with a pharmaceutically acceptable diluent, excipient, or carrier. The extracts or chemical compositions may be added to a carrier in the form of a salt or solvate. For example, in cases where extracts or chemical compositions are sufficiently basic or acidic to form stable nontoxic acid or base salts, administration of the extracts or chemical compositions as salts may be appropriate. Examples of pharmaceutically acceptable salts are organic acid addition salts formed with acids that form a physiological acceptable anion, for example, tosylate, methanesulfonate, acetate, citrate, malonate, tartrate, succinate, benzoate, ascorbate, α-ketoglutarate, and β-glycerophosphate. Suitable inorganic salts may also be formed, including hydrochloride, halide, sulfate, nitrate, bicarbonate, and carbonate salts.

Pharmaceutically acceptable salts may be obtained using standard procedures well known in the art, for example by reacting a sufficiently basic extract or chemical composition, such as an amine, with a suitable acid to provide a physiologically acceptable ionic extract or chemical composition. Alkali metal (for example, sodium, potassium or lithium) or alkaline earth metal (for example, calcium) salts of carboxylic acids can also be prepared by analogous methods.

The extracts or chemical compositions described herein can be formulated as pharmaceutical compositions and administered to a mammalian host, such as a human patient, in a variety of forms. The forms can be specifically adapted to a chosen route of administration, e.g., oral or parenteral administration, by intravenous, intramuscular, topical or subcutaneous routes. The extracts or chemical compositions described herein may be systemically administered in combination with a pharmaceutically acceptable vehicle, such as an inert diluent or an assimilable edible carrier. They may be formulated with a cyclodextrin, especially if taste masking is a desired feature of the product. For oral administration, extracts or chemical compositions can be enclosed in hard or soft shell gelatin capsules, compressed into tablets, or incorporated directly into the food of a patient's diet. Extracts or chemical compositions may also be combined with one or more excipients and used in the form of ingestible tablets, buccal tablets, troches, capsules, elixirs, suspensions, syrups, wafers, films, and the like. Such compositions and preparations typically contain at least 0.1% of active extract or chemical composition. The percentage of active ingredient in the compositions and preparations can vary and may conveniently be from about 0.5% to about 60%, about 1% to about 25%, or about 2% to about 10%, of the weight of a given unit dosage form. The amount of active extract or chemical composition in such therapeutically useful compositions can be such that an effective dosage level can be obtained.

The tablets, troches, pills, capsules, and the like may also contain one or more of the following: binders such as gum tragacanth, acacia, corn starch or gelatin; excipients such as dicalcium phosphate; a disintegrating agent such as corn starch, potato starch, alginic acid and the like; and a lubricant such as magnesium stearate. A sweetening agent such as sucrose, fructose, lactose or aspartame; or a flavoring agent such as peppermint, oil of wintergreen, or cherry flavoring, may be added. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propyl parabens as preservatives, a dye and flavoring such as cherry or orange flavor. Any material used in preparing any unit dosage form should be pharmaceutically acceptable and substantially non-toxic in the amounts employed. In addition, the active ingredient may be incorporated into sustained-release preparations and devices.

The active ingredient may be administered intravenously or intraperitoneally by infusion or injection. Solutions of the active ingredient or its salts can be prepared in water, optionally mixed with a nontoxic surfactant. Dispersions can be prepared in glycerol, liquid polyethylene glycols, triacetin, or mixtures thereof, or in a pharmaceutically acceptable oil. Under ordinary conditions of storage and use, preparations may contain a preservative to prevent the growth of microorganisms.

Pharmaceutical dosage forms suitable for injection or infusion can include sterile aqueous solutions, dispersions, or sterile powders comprising the active ingredient adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions, or by the use of surfactants. The prevention of the action of microorganisms can be brought about by various antibacterial and/or antifungal agents, for example, parabens, chlorobutanol, phenol, sorbic acid, thimerosal, and the like. In many cases, it will be preferable to include isotonic agents, for example, sugars, buffers, or sodium chloride. Prolonged absorption of the injectable compositions can be brought about by agents delaying absorption, for example, aluminum monostearate and/or gelatin.

Sterile injectable solutions can be prepared by incorporating the active ingredient in the required amount in the appropriate solvent with various other ingredients enumerated above, as required, optionally followed by filter sterilization. In the case of sterile powders for the preparation of sterile injectable solutions, methods of preparation can include vacuum drying and freeze drying techniques, which yield a powder of the active ingredient plus any additional desired ingredient present in the solution.

For topical administration, extracts or chemical compositions may be applied in pure form, e.g., when they are liquids. However, it will generally be desirable to administer the active agent to the skin as a composition or formulation, for example, in combination with a dermatologically acceptable carrier, which may be a solid, a liquid, a gel, or the like.

Useful solid carriers include finely divided solids such as talc, clay, microcrystalline cellulose, silica, alumina, and the like. Useful liquid carriers include water, dimethyl sulfoxide (DMSO), alcohols, glycols, or water-alcohol/glycol blends, in which a compound can be dissolved or dispersed at effective levels, optionally with the aid of non-toxic surfactants. Adjuvants such as fragrances and additional antimicrobial agents can be added to optimize the properties for a given use. The resultant liquid compositions can be applied from absorbent pads, used to impregnate bandages and other dressings, or sprayed onto the affected area using a pump-type or aerosol sprayer.

Thickeners such as synthetic polymers, fatty acids, fatty acid salts and esters, fatty alcohols, modified celluloses, or modified mineral materials can also be employed with liquid carriers to form spreadable pastes, gels, ointments, soaps, and the like, for application directly to the skin of the user.

Examples of dermatological compositions for delivering active agents to the skin are known to the art; for example, see U.S. Patent Nos. 4,992,478 (Geria), 4,820,508 (Wortzman), 4,608,392 (Jacquet et al.), and 4,559,157 (Smith et al.). Such dermatological compositions can be used in combinations with the extracts or chemical compositions described herein where an ingredient of such compositions can optionally be replaced by a compound described herein, or an extract or chemical composition described herein can be added to the composition

Useful dosages of the extracts or chemical compositions described herein can be determined by comparing their *in vitro* and/or *in vivo* activities in animal models. Methods for the extrapolation of effective dosages in mice, and other animals, to humans are known to the art; for example, see U.S. Patent No. 4,938,949 (Borch et al.). The amount of an extract or chemical composition, or an active salt or derivative thereof, required for use in treatment will vary not only with the particular extract, chemical composition or salt thereof selected, but also with the route of administration, the nature of the condition being treated, and the age and condition of the patient, and will be ultimately at the discretion of an attendant physician or clinician.

The extracts or chemical compositions described herein can be conveniently administered in a unit dosage form, for example, containing 5 to 1000 mg/m², conveniently 10 to 750 mg/m², most conveniently, 50 to 500 mg/m² of active ingredient per unit dosage form. The desired dose may conveniently be presented in a single dose or as divided doses administered at appropriate intervals, for example, as two, three, four or more sub-doses per day. The sub-dose itself may be further divided, e.g., into a number of discrete loosely spaced administrations.

The extracts or chemical compositions described herein can be useful for therapeutic methods of treating a cancer in a mammal, which involve administering to a mammal having cancer an effective amount of an extract or chemical composition described herein. A mammal includes a primate, human, rodent, canine, feline, bovine, ovine, equine, swine, caprine, bovine and the like. Cancer refers to any various type of malignant neoplasm, for example, colon cancer, breast cancer, melanoma and leukemia, and in general is characterized by an undesirable cellular proliferation, e.g., unregulated growth, lack of differentiation, local tissue invasion, and metastasis.

The ability of an extract or chemical composition described herein to treat cancer may be determined by using assays well known to the art. For example, the design of treatment protocols, toxicity evaluation, data analysis, quantification of tumor cell kill, and the biological significance of the use of transplantable tumor screens are known.

The following Examples are intended to illustrate the above invention and should not be construed as to narrow its scope. One skilled in the art will readily recognize that the Examples suggest many other ways in which the invention could be practiced. It should be understood that numerous variations and modifications may be made while remaining within the scope of the invention.

### General Procedure

### Stage 1 - winterization

About 125 lbs. of fresh cut cannabis plant material were placed in a sealable vessel (e.g., glass vessel) in a dark environment. About 180 lbs. of butane (about 1.5 times the volume of plant material) were added to the vessel to cover the plant material. The plant material was allowed to soak in the butane for up to about two hours to macerate it. This is the first solution. The first solution was filtered to eliminate undesirable green vegetable matter, which produced a concentrated extract (e.g., butane hash oil or botanical drug substance). This was the first filtrate. The first filtrate was then added to a vessel containing about 125-180 lbs. of ethanol (about 1.0 to 1.5 times the volume of filtrate). This was the second solution. The butane was evaporated off with the help of an exhaust fan and the second solution was put in a freezer or ice bath to chill it for about twenty-four hours. The chilled second solution was then filtered to remove any undesirable solid matter. This was the second filtrate. The ethanol was evaporated off to produce a concentrate of cannabis plant material substantially free of undesirable chemicals.

### Stage 2 - (a) hot temperature distillation versus (b) ambient temperature distillation

The concentrate was allowed to warm to room temperature and then was put into a vacuum drying oven connected to a sealable single-chamber cold trap system like the one that is shown in the drawings. In the hot temperature distillation (a), the drying oven was set at about 300 °F. In the ambient temperature distillation (b), the drying oven was kept ar room temperature, around 65 °F.

### Stage 3 - cold trap condensation

A vacuum was connected to the system to apply negative pressure and the concentrate was volatilized at a low temperature and transported to the sealable chamber, where it was condensed to form a desired extract, which was then collected in a bowl. The collected extract was mixed with ice. The ice mixture of plant extract was allowed to melt and the resulting fluid was poured into a separatory funnel and allowed to sit for about twenty-four hours at room temperature to effect separation. Two layers were separated in the funnel. The top layer was amber (or golden) colored and the bottom layer was clear. The two layers were drawn out via a valve at the bottom of the funnel and stored in separate vessels. The two layers comprised different chemical compositions that are enriched with desirable terpenes, terpenoids and other chemical compounds. We tested the amber layers using the (a) hot temperature distillation (300 °F) versus the (b) ambient temperature distillation (65 °F).

### Examples 1 and 2

The general procedures were adapted and modified for several experimental runs of the processes disclosed herein, which produced a series of extracts. Comparative data is provided for the (a) hot temperature distillation system of Example 1 (about 300 °F) and the (b) ambient temperature distillation system of Example 2 (about 65 °F).

### Stage 1 - winterization

About 10 lbs. of undried cut cannabis plant material were placed in a 10 gallon sealable glass vessel in a dark environment. About 5 gallons of butane were added to the vessel to cover the plant material. The submerged plant material was allowed to soak in the butane for about 30 minutes to macerate it. This is the first solution. The first solution was filtered through a coffee filter to eliminate undesirable green vegetable matter, which produced a concentrated extract. This was the first filtrate. The first filtrate was then mixed in a 10 gallon sealed vessel with two gallons of ethanol. This was the second solution. The container was allowed to sit for a period of time to evaporate off solvent. Butane evaporation was promoted with the help of an exhaust fan. After evaporation, the second solution was put in a freezer at around 32 °F and allowed to sit for about seventy-two hours. The chilled second solution was then filtered through a coffee filter into an evaporation dish to remove any undesirable solid matter. This was the second filtrate. Ethanol evaporation was promoted with the help of an exhaust fan. After about 2 days, the ethanol and any water content were evaporated off to produce a concentrate of cannabis plant material substantially free of undesirable chemicals.

### Stage 2 - distillation and condensation extraction process

The resulting concentrates from Stage 1 were put into a vacuum oven at a temperature of about 300 °F for Example 1 and at a temperature of about 65 °F for Example 2. Example 1 is a conventional hot temperature distillation subjected to a cold trap condensation. Example 2 is an ambient temperature distillation subjected to a cold trap condensation.

### Stage 3 - cold trap condensation

A vacuum level of about 600 millitorr was achieved utilizing a Laybold two-stage vacuum pump with fresh oil. A cold trap system cooled with liquid nitrogen was placed in line between the drying oven and the vacuum pump, and was maintained at about -270 °F. After about two hours, the vacuum pump was turned off. The system was de-pressurized and the frozen contents deposited in the cold trap were removed in the form of ice. The ice was allowed to melt in a sealed container, then poured into a separatory funnel. After about one hour, the contents of the separatory funnel settled out and formed two distinct layers. The top layer was amber (golden) in color and contained desirable terpenes and terpene derivatives. The bottom layer was relatively clear and likely contained a hydrosol. Via a valve, the two layers were drawn out and separated. The amber layers of Examples 1 and 2 were subjected to characterization testing disclosed below.

### Comparative Characterization Data

Several runs of Example 1 and Example 2 were conducted. The following tables show the characterization data that were generated for the sample testing of amber extracts of Example 1 and Example 2. The following thirty-four terpenes and terpene derivatives were identified in one or more samples.

**TABLE 1**

| Compounds Identified in Condensation Samples of Example 1 (hot temperature distillation) and Example 2 (ambient temperature distillation. | | | | |
|---|---|---|---|---|
| No. | | Name | No. | Name |
| 1 | Aristolene epoxide | | 18 | d-Limonene |
| 2 | cis-α-Bisabolene | | 19 | Linalool |
| 3 | α-Bisabolol | | 20 | Linalool oxide |
| 4 | Camphene | | 21 | β -Myrcene |
| 5 | δ-3-Carene | | 22 | Nerolidol |
| 6 | β-Caryophyllene | | 23 | Ocimene |
| 7 | Caryophyllene oxide | | 24 | Phytol |
| 8 | p-Cymene | | 25 | trans-Pinalol |
| 9 | Eucalyptol | | 26 | α-Pinene |
| 10 | Eudesma-3,7(11)-diene | | 27 | β-Pinene |
| 11 | β -Farnesene | | 28 | iso-Pulegol |
| 12 | exo-Fenchol | | 29 | α-Selenene |
| 13 | Geraniol | | 30 | α-Terpinene |
| 14 | Guaiol | | 31 | γ-Terpinene |
| 15 | τ-Gurjunene | | 32 | α-Terpineol |
| 16 | α-Humulene (α-Caryophyllene) | | 33 | Terpinolene |
| | | | 34 | Ylangene |
| 17 | iso-Borneol | | | |

**TABLE 2**

| (values are mg/ml) | | | | |
|---|---|---|---|---|
| Compound No. | | Example 1 | | Example 2 |
| | 6 | | 0.063 | 9.1 |
| | 7 | | 0.00 | 2.0 |
| | 16 | | 0.00 | 5.1 |
| | 19 | | 0.052 | 1.1 |
| | 21 | | 0.00 | 9.5 |
| | 26 | | 0.00 | 5.9 |
| | 33 | | 0.00 | 1.8 |

**TABLE 3**

| (values are mg/g) | | | |
|---|---|---|---|
| Compound No. | | Example 1 | Example 2 |
| | 3 | 0.00 | 0.06 |
| | 4 | 0.01 | 0.06 |
| | 5 | 0.00 | 0.01 |
| | 6 | 0.00 | 0.07 |
| | 7 | 0.00 | 4.57 |
| | 8 | 0.00 | 0.04 |
| | 9 | 0.00 | 0.02 |
| | 13 | 0.00 | 0.12 |
| | 14 | 0.00 | 0.01 |
| | 16 | 0.00 | 0.12 |
| | 18 | 0.00 | 0.90 |
| | 19 | 0.00 | 0.05 |
| | 21 | 0.01 | 0.30 |
| | 22 | 0.00 | 0.01 |
| | 23 | 0.00 | 0.02 |
| | 26 | 0.00 | 0.23 |
| | 27 | 0.00 | 0.07 |
| | 28 | 0.00 | 0.02 |
| | 30 | 0.00 | 0.23 |
| | 31 | 0.00 | 0.01 |
| | 33 | 10.00 | 15.96 |

Compounds 7, 13, 16, 18, 21, 26, 20 and 33 were all identified at greater than 0.12 mg/g for Example 2.

**TABLE 4**

| [four terpene fractions] (values are percentage of total peak area from total ion chromatograms) | | | | |
|---|---|---|---|---|
| Compound No. | Sample A | Sample B | Sample C | Sample D |
| 1 | 0.0 | 0.0 | 0.0 | 0.7 |
| 2 | 0.0 | 0.0 | 0.0 | 2.7 |
| 4 | 0.0 | 0.0 | 0.0 | 1.2 |
| 6 | 6.2 | 27.0 | 13.8 | 17.8 |
| 7 | 0.0 | 1.1 | 7.8 | 1.5 |
| 10 | 0.0 | 7.7 | 2.6 | 3.5 |
| 11 | 1.4 | 0.0 | 0.0 | 0.0 |
| 12 | 17.5 | 4.1 | 10.2 | 3.7 |
| 15 | 0.0 | 1.7 | 1.2 | 1.0 |
| 16 | 1.6 | 9.5 | 5.3 | 6.8 |
| 17 | 3.6 | 0.0 | 1.9 | 0.7 |
| 18 | 0.0 | 5.9 | 5.3 | 11.4 |
| 19 | 31.1 | 6.6 | 15.3 | 7.1 |
| 20 | 0.0 | 0.0 | 0.7 | 0.0 |
| 21 | 0.0 | 2.2 | 1.7 | 8.6 |
| 25 | 0.0 | 2.5 | 4.1 | 2.4 |
| 27 | 0.0 | 1.7 | 1.6 | 4.2 |
| 27 | 0.0 | 2.0 | 1.4 | 5.5 |
| 29 | 0.0 | 3.5 | 0.0 | 0.0 |
| 32 | 14.9 | 1.6 | 3.6 | 1.6 |
| 34 | 0.0 | 1.6 | 1.3 | 1.2 |

The clear layer extracts generated during the runs appear to be hydrosols that contained relatively low amounts of desirable terpenes and/or terpene derivatives. On the other hand, the amber layer extracts may be an essence oil or something similar to an essence oil, and contained high concentrations of desirable terpenes and terpene derivatives. As can be seen in the tables, the conventional extracts of Example 1 contained lower concentrations of desirable terpenes and terpene derivatives, and did not contain some of the terpenes and terpene derivatives found in the extracts of Example 2. The lower and/or missing concentrations of desirable terpenes and terpene derivatives found in the conventional extracts of Example 1 were likely due to the application of heat, which degraded or destroyed desirable chemicals, particularly, those chemicals of lower molecular weight.

While specific embodiments have been described above with reference to the disclosed embodiments and examples, such embodiments are only illustrative and do not limit the scope of the invention. Changes and modifications can be made in accordance with ordinary skill in the art without departing from the invention in its broader aspects as defined in the following claims.

All publications, patents, and patent documents are incorporated by reference herein, as though individually incorporated by reference. No limitations inconsistent with this disclosure are to be understood therefrom. The invention has been described with reference to various specific and preferred embodiments and techniques. However, it should be understood that many variations and modifications may be made while remaining within the spirit and scope of the invention.

### Embodiments

1. A method of producing an extract from a plant material, the method comprising:
   (a) placing the plant material in a compartment at a temperature of less than about 35 °C;
   (b) applying positive or negative pressure to the compartment to volatilize chemical compounds contained in the plant material at a temperature of less than about 35 °C;
   (c) transporting the volatilized chemical compounds via a conduit to a condenser comprising at least one sealable chamber;
   (d) condensing the volatilized chemical compounds in the condenser to form the extract; and
   (e) collecting the extract from the condenser.
2. The method of embodiment 1, where a vacuum apparatus is used to apply negative pressure to the compartment.
3. The method of embodiment 1, where the plant material comprises cannabis.
4. The method of embodiment 1, where the plant material is wet or dry.
5. The method of embodiment 1, where prior to step (a), the plant material was previously subjected to one or more solvent extractions, supercritical fluid extractions, fractional supercritical fluid extractions, sub-critical fluid extractions, fractional sub-critical fluid extractions, or a combination thereof
6. The method of embodiment 5, where the solvent or fluid comprises (i) a polar protic solvent system, (ii) a polar aprotic solvent system, (iii) a nonpolar protic solvent system, (iv) a nonpolar aprotic solvent system, or (v) a combination thereof.
7. The method of claim 5, where the solvent or fluid comprises carbon dioxide, hydrogen, neon, nitrogen, argon, methane, ethane, propane, ammonia, water, xenon, methanol, ethanol, 1-propanol, 2-propanol, 1-hexanol, 2-methoxy ethanol, tetrahydrofuran, 1,4-dioxane, acetonitrile, methylene chloride, dichloroethane, chloroform, ethyl acetate, propylene carbonate, N,N-dimethylacetamide, dimethyl sulfoxide, formic acid, carbon disulfide, acetone, toluene, hexanes, pentanes, trifluoromethane, nitrous oxide, sulfur hexafluoroide, butane, isobutane, ethyl ether, benzotrifluoride, (p-chlorophenyl)
   trifluoromethane, chlorofluorocarbon (CFC), hydrofluorocarbon, HFA-134a, terpenes, terpenoids, or a mixture thereof.
8. The method of embodiment 1, where prior to step (a), the plant material was previously winterized.
9. The method of embodiment 1, where the condenser comprises at least two cooling chambers of differing temperatures to produce at least two different extracts.
10. The method of embodiment 1, wherein the volatilization step (b) is conducted in association with rotary evaporation, microwave radiation, ultrasonic waves, convection heat, and/or conduction heat.
11. The method of embodiment 1, where the extract comprises chemical compounds, solvent, and ice, the process further comprising melting the ice and separating the chemical compounds, solvent, and water from one another.
12. The method of embodiment 11, further comprising separating the chemical compounds into at least two different compositions.
13. The method of embodiment 1, where the condensation step (d) is carried out at or below near-freezing temperature.
14. A cannabis plant material extract comprising, β-caryophyllene, caryophyllene oxide, ct-humulene ( -caryophyllene), linalool, β-myrcene, α-pinene, and terpinolene.
15. A cannabis plant material extract comprising, β-caryophyllene, caryophyllene oxide, geraniol, ct-humulene (a-caryophyllene), d-limonene, β-myrcene, α-pinene, α-terpinene, and terpinolene.
16. A cannabis plant material extract comprising:
   (i) β-caryophyllene, exo-fenchol, linalool and oo-terpineol;
   (ii) β-caryophyllene, eudesma-3,7(l l)-diene, a-humulene, d-limonene and linalool;
   (iii) β-caryophyllene, caryophyllene oxide, exo-fenchol and linalool; or
   (iv) β-caryophyllene, a-humulene, d-limonene, linalool and β-myrcene.
17. A pharmaceutical composition, fragrance or flavoring comprising an extract produced by the method of embodiment 1.
18. An apparatus for producing an extract from a plant material, the apparatus comprising a compartment capable of volatilizing the plant material via positive or negative pressure at a temperature of less than about 35 °C, the compartment being connected to a conduit capable of transporting volatilized plant material to a condenser capable of maintaining cool or cold temperatures and comprising at least one sealable chamber that contains or is connected to at least one collector having non-absorbent, absorbent, non-adsorbent, or adsorbent properties, the sealable chamber having a bottom, an air intake, an air outtake, and optionally, a valve at the bottom.
19. The apparatus of embodiment 18, where the condenser comprises a plurality of plates, coils, and/or baffles.
20. The apparatus of embodiment 18, where the plant material comprises cannabis.
21. A method of producing an extract from cannabis, the method comprising:
   (a) placing the cannabis in a compartment that is at a temperature of less than about 35°C;
   (b) applying negative pressure, via a vacuum apparatus in fluid communication with the compartment, a conduit and a condenser, to the compartment to volatilize one or more terpenes contained in the cannabis at a temperature of less than about 35°C;
   (c) transporting, while the vacuum apparatus is applying negative pressure to the compartment, the conduit and the condenser, the one or more volatilized terpenes via the conduit to the condenser comprising at least one sealable chamber;
   (d) condensing, while the vacuum apparatus is applying negative pressure to the compartment, the conduit and the condenser, the one or more volatilized terpenes in the condenser to form the extract, where the condensation step (d) is carried out at or below 0°C; and
   (e) collecting the extract comprising terpenes from the condenser.
22. The method of embodiment 22, further comprising the step of subjecting the cannabis to one or more solvent extractions, supercritical fluid extractions, fractional supercritical fluid extractions, sub-critical fluid extractions, fractional sub-critical fluid extractions, or a combination thereof, prior to step (a).
23. The method of embodiment 22, where the solvent or fluid comprises (i) a polar protic solvent system, (ii) a polar aprotic solvent system, (iii) a nonpolar protic solvent system, (iv) a nonpolar aprotic solvent system, or (v) a combination thereof; or
   where the solvent or fluid comprises carbon dioxide, hydrogen, neon, nitrogen, argon, methane, ethane, propane, ammonia, water, xenon, methanol, ethanol, 1-propanol, 2-propanol, 1-hexanol, 2-methoxy ethanol, tetrahydrofuran, 1,4-dioxane, acetonitrile, methylene chloride, dichloroethane, chloroform, ethyl acetate, propylene carbonate, *N,N-*dimethylacetamide, dimethyl sulfoxide, formic acid, carbon disulfide, acetone, toluene, hexanes, pentanes, trifluoromethane, nitrous oxide, sulfur hexafluoroide, butane, isobutane, ethyl ether, benzotrifluoride, (p-chlorophenyl) trifluoromethane, chlorofluorocarbon (CFC), hydrofluorocarbon, HFA-134a, or a mixture thereof.
24. The method of embodiment 22 or 23, further comprising the step of winterizing the cannabis prior to step (a).
25. The method of embodiment 21, where the condenser comprises at least two cooling chambers of differing temperatures, thus enabling collection of at least two different extracts.
26. The method of embodiment 21, wherein the volatilization step (b) is conducted in association with rotary evaporation, microwave radiation, ultrasonic waves, convection heat, and/or conduction heat.
27. The method of embodiment 22, where the extract comprises terpenes, solvent, and ice, the process further comprising melting the ice and separating the terpenes, solvent, and water from one another.
28. The method of embodiment 25, where the condenser comprises three cooling chambers of differing temperatures, the first cooling chamber having the least cooled temperature and the second and third chambers having progressively cooler temperatures.
29. The method of any of embodiments 21 to 28, where the extract includes at least one of the following compounds: cannabinol, cannabinolic acid, DELTA 9-tetrahydrocannabinol, DELTA 9-tetrahydrocannabinolic acid, DELTA 9-cannabidiol, DELTA 9-tetrahydrocannabidiolic acid, DELTA 8-tetrahydrocannabinol, DELTA 8-tetrahydrocannabinolic acid, DELTA 8-tetrahydrocannabidiol, DELTA 8-tetrahydrocannabidiolic acid, DELTA 9-tetrahydrocannabivarin, cannabigerol, cannabigerolic acid, cannabichromene, cannabichromenic acid, cannabicyclol, cannabicyclolic acid, cannabivarin, tetrahydrocannabivarin, cannabidivarin, cannabichromevarin, cannabigerovarin, cannabigerol monomethyl ether, beta-caryophyllene epoxide, mentha-1,8(9)-dien-5-ol, pulegone, limonene, limonene oxide, alpha-terpinene, terpinen-4-ol, carvacrol, carvone, 1,8-cineole, p-cymene, fenchone, pulegone-1,2epoxide, beta-myrcene, cannflavin A, and cannflavin B.
30. The method of any one of embodiments 21 and 25 to 29, where the placing step (a) comprises placing undried cannabis plant material in the compartment.
31. The method of any one of embodiments 21 and 25 to 29, where the placing step (a) comprises placing dried cannabis plant material in the compartment.
32. The method of any one of embodiments 21 to 29, where the placing step (a) comprises placing a concentrate of cannabis plant material in the compartment.

## Claims

1. A method of producing an extract comprising terpenes from a plant material that includes terpenes, the method comprising:
(a) placing the plant material in a compartment;
(b) applying negative pressure, via a vacuum apparatus in fluid communication with the compartment, a conduit and a condenser, to the compartment to volatilize one or more terpenes contained in the plant material at a temperature of less than about 35 °C;
(c) transporting while the vacuum apparatus is applying negative pressure to the compartment, the conduit and the condenser, the one or more volatilized terpenes via a conduit to a condenser comprising at least one sealable chamber;
(d) condensing, while the vacuum apparatus is applying negative pressure to the compartment, the conduit and the condenser, the one or more volatilized terpenes in the condenser to form the extract; and
(e) collecting the extract comprising terpenes from the condenser.

2. The method of claim 1, where prior to step (a), the plant material is subjected to one or more solvent extractions, supercritical fluid extractions, fractional supercritical fluid extractions, sub-critical fluid extractions, fractional sub-critical fluid extractions, or a combination thereof.

3. The method of claim 5, where the solvent or fluid comprises (i) a polar protic solvent system, (ii) a polar aprotic solvent system, (iii) a nonpolar protic solvent system, (iv) a nonpolar aprotic solvent system, or (v) a combination thereof; or where the solvent or fluid comprises carbon dioxide, hydrogen, neon, nitrogen, argon, methane, ethane, propane, ammonia, water, xenon, methanol, ethanol, 1-propanol, 2-propanol, 1-hexanol, 2-methoxy ethanol, tetrahydrofuran, 1,4-dioxane, acetonitrile, methylene chloride, dichloroethane, chloroform, ethyl acetate, propylene carbonate, N,N-dimethylacetamide, dimethyl sulfoxide, formic acid, carbon disulfide, acetone, toluene, hexanes, pentanes, trifluoromethane, nitrous oxide, sulfur hexafluoroide, butane, isobutane, ethyl ether, benzotrifluoride, (p-chlorophenyl)trifluoromethane, chlorofluorocarbon (CFC), hydrofluorocarbon, HFA-134a, or a mixture thereof.

4. The method of any preceding claim, where prior to step (a), the plant material is winterized.

5. The method of claim 1, where the extract comprises terpenes, solvent, and ice, the method further comprising melting the ice and separating the terpenes, solvent, and water from one another.

6. The method of claim 1, where the condenser comprises at least two cooling chambers of differing temperatures, thus enabling collection of at least two different extracts.

7. The method of claim 6, with the condenser comprising three cooling chambers of differing temperatures, a first cooling chamber having the least cooled temperature, and second and third cooling chambers having progressively cooler temperatures.

8. The method of claim 1, wherein the volatilization step (b) is conducted in association with rotary evaporation, microwave radiation, ultrasonic waves, convection heat, and/or conduction heat.

9. The method of claim 1, where the condensation step (d) is carried out at or below 0°C.

10. The method of any preceding claim, wherein the plant material is a concentrate.

11. The method of claim 1, where the placing step (a) comprises placing undried plant material in the compartment.

12. The method of claim 1, where the placing step (a) comprises placing dried plant material in the compartment.

13. The method of any preceding claim, where the plant material comprises cannabis.

14. The method of claim 13, where the extract includes at least one of the following compounds:
cannabinol, cannabinolic acid, DELTA 9-tetrahydrocannabinol, DELTA 9-tetrahydrocannabinolic acid, DELTA 9-cannabidiol, DELTA 9-tetrahydrocannabidiolic acid, DELTA 8-tetrahydrocannabinol, DELTA 8-tetrahydrocannabinolic acid, DELTA 8-tetrahydrocannabidiol, DELTA 8-tetrahydrocannabidiolic acid, DELTA 9-tetrahydrocannabivarin, cannabigerol, cannabigerolic acid, cannabichromene, cannabichromenic acid, cannabicyclol, cannabicyclolic acid, cannabivarin, tetrahydrocannabivarin, cannabidivarin, cannabichromevarin, cannabigerovarin, cannabigerol monomethyl ether, beta-caryophyllene epoxide, mentha-1,8(9)-dien-5-ol, pulegone, limonene, limonene oxide, alpha-terpinene, terpinen-4-ol, carvacrol, carvone, 1,8-cineole, p-cymene, fenchone, pulegone-1,2epoxide, beta-myrcene, cannflavin A, and cannflavin B in the condenser to form the extract.
